(19) Europäisches Patentamt
European Patent Office
Office européen des brevets

(11) EP 4 657 303 A1

(12) **EUROPEAN PATENT APPLICATION**
published in accordance with Art. 153(4) EPC

(43) Date of publication:
**03.12.2025 Bulletin 2025/49**

(21) Application number: **24823261.3**

(22) Date of filing: **03.06.2024**

(51) International Patent Classification (IPC):
***G06F 30/20*** *(2020.01)*

(52) Cooperative Patent Classification (CPC):
**G06F 30/20**

(86) International application number:
**PCT/JP2024/020252**

(87) International publication number:
**WO 2024/257641 (19.12.2024 Gazette 2024/51)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **13.06.2023 JP 2023097295**

(71) Applicant: **JFE Steel Corporation**
**Tokyo 100-0011 (JP)**

(72) Inventors:
- **TAKEHARA, Marina**
  **Tokyo 100-0011 (JP)**
- **TAKASHITA, Takuya**
  **Tokyo 100-0011 (JP)**
- **NAKAMURA, Takechika**
  **Tokyo 100-0011 (JP)**
- **HIRATANI, Tatsuhiko**
  **Tokyo 100-0011 (JP)**

(74) Representative: **Hoffmann Eitle**
**Patent- und Rechtsanwälte PartmbB**
**Arabellastraße 30**
**81925 München (DE)**

(54) **IRON LOSS EVALUATION METHOD, LOW IRON LOSS MATERIAL DESIGN METHOD, IRON LOSS EVALUATION PROGRAM, LOW IRON LOSS MATERIAL DESIGN PROGRAM, LOW LOSS SOFT MAGNETIC MATERIAL, AND LOW LOSS SOFT MAGNETIC POWDER**

(57) A method of evaluating iron loss includes predicting, based on a modulation factor and a carrier frequency, iron loss that occurs when a magnetic material is excited by an excitation waveform generated by a PWM inverter that is controlled using PWM parameters including the modulation factor and the carrier frequency.

FIG. 4

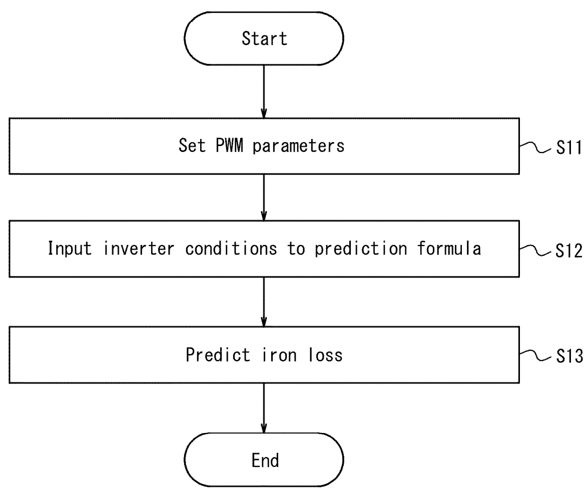


EP 4 657 303 A1

## Description

### TECHNICAL FIELD

The present disclosure relates to a method and program for evaluating iron loss, which becomes a problem when exciting a magnetic material with a PWM inverter, a method and program for designing a low iron loss material that corresponds to the excitation conditions of a PWM inverter, and a low loss soft magnetic material and low loss soft magnetic powder usable as a low iron loss material.

### BACKGROUND

In general, the iron loss of soft magnetic material used as the iron core of a motor or the like is evaluated by exciting the material with a sine wave. On the other hand, the material of the iron core of motors and the like used in recent power electronic devices is excited by an inverter. Here, iron loss causes heat generation in power electronic devices. In order to improve the accuracy of predictions of the amount of heat generated during the actual operation of power electronic devices, it is essential to evaluate the iron loss when the iron core is excited by an inverter. As a method of evaluating iron loss when an iron core is excited by an inverter, a method is known in which the iron loss is calculated by integrating an excitation waveform synthesized based on inverter conditions (see Non-patent Literature (NPL) 1).

Here, a PWM (Pulse Width Modulation) inverter, which is the most common type of inverter, is sometimes used. The PWM inverter generates an excitation waveform as a pulse wave by the PWM method. Specifically, in order to create a sine wave with a maximum magnetic flux density of B1 and a frequency of f1 as the target excitation wave, the PWM inverter compares the height of a modulated wave corresponding to the target excitation wave with the height of a carrier wave, which is a triangular wave having a frequency several times that of the modulated wave, and generates a pulse wave that is at a HI level in a timeslot when the modulated wave is greater than the carrier wave and at a LO level in a timeslot when the modulated wave is smaller than the carrier wave. This pulse wave is configured so that the pulse height is constant regardless of time, and the pulse width changes with time. The time-averaged waveform of the pulse wave thus generated is applied to the iron core as an excitation wave.

As described above, the excitation wave generated by the PWM inverter corresponds to a waveform in which harmonics caused by a carrier wave are superimposed on a sine wave, which is the target excitation wave. The iron loss in excitation by a PWM inverter is increased by the amount of iron loss caused by harmonic components, as compared to the iron loss in excitation by a sine wave.

Therefore, it is conceivable to improve the accuracy of iron loss calculation by analyzing the excitation wave generated by the PWM inverter, but a complex waveform analysis is required to analyze the excitation wave generated by the PWM inverter. Therefore, when prototyping iron cores under a plurality of conditions, it is difficult to perform waveform analysis for each of the conditions. To address this issue, a method of estimating iron loss quickly and accurately has been proposed (see Patent Literature (PTL) 1).

### CITATION LIST

#### Patent Literature

PTL 1: JP 2012-26960 A

#### Non-patent Literature

NPL 1: G. Bertotti, IEEE TRANSACTIONS ON MAGNETICS, VOL. 24, NO. 1 (1988), pp. 621-630

### SUMMARY

#### (Technical Problem)

As described in NPL 1, the method of evaluating inverter iron loss is extremely complicated, as it involves synthesizing an excitation waveform based on inverter conditions, and then integrating the synthesized waveform. This poses an obstacle to the analysis itself, the prototyping of magnetic cores, and their application to evaluation. Furthermore, in PTL 1, the harmonic components of the inverter excitation waveform are expressed in a simplified manner to estimate the iron loss, but it is necessary to measure the harmonic components for each iron loss estimation.

The present disclosure has been developed in consideration of the above-described problems and aims to provide a method of evaluating iron loss and an iron loss evaluation program that clarify the relationship between inverter input conditions and inverter iron loss, and that can directly predict iron loss from inverter conditions without measuring iron loss when an iron core is excited by an inverter. In addition, the present disclosure aims to provide a method of designing a low iron loss material and a low iron loss material design program using evaluation results from the above-described method of evaluating iron loss and iron loss evaluation program, as well as a low loss soft magnetic material and a low loss soft magnetic powder designed using the method of designing a low iron loss material and low iron loss material design program.

(Solution to Problem)

(1) A method of evaluating iron loss according to an embodiment of the present disclosure includes predicting, based on a modulation factor and a carrier frequency, iron loss that occurs when a magnetic material is excited by an excitation waveform generated by a PWM inverter that is controlled using PWM parameters including the modulation factor and the carrier frequency.

(2) In the predicting of the iron loss in the method of evaluating iron loss of (1), the iron loss may be predicted based on a ratio of a frequency of a harmonic of the excitation waveform to the carrier frequency.

(3) In the predicting of the iron loss in the method of evaluating iron loss of (2), a ratio of a frequency of a harmonic of the excitation waveform to the carrier frequency may be determined based on waveform data obtained by measuring the excitation waveform generated by the PWM inverter.

(4) In the predicting of the iron loss in the method of evaluating iron loss of any one of (1) to (3), when the magnetic material is a dust core, the iron loss may be predicted further based on a cross-sectional area of the dust core that intersects with a magnetic flux that excites the dust core.

(5) A method of designing low iron loss material according to an embodiment of the present disclosure includes designing a characteristic of a magnetic material to be designed so as to reduce iron loss occurring when the magnetic material to be designed is excited by an excitation waveform generated by controlling a PWM inverter at a predetermined modulation factor and a predetermined carrier frequency, based on a result of executing the method of evaluating iron loss in any one of (1) to (4) above to predict iron loss occurring when each of at least two types of magnetic materials having different characteristics is excited by an excitation waveform generated by controlling the PWM inverter at the predetermined modulation factor and the predetermined carrier frequency.

(6) In the designing of the characteristic of the magnetic material to be designed in the method of designing low iron loss material in (5), a representative dimension of the magnetic material to be designed may be designed as the characteristic of the magnetic material to be designed based on a result of acquiring, for each representative dimension of the magnetic material to be designed, the iron loss generated when the magnetic material to be designed is excited by a sinusoidal excitation waveform.

(7) In the method of designing low iron loss material in (5), the magnetic material to be designed may be a dust core made of soft magnetic powder. In the designing of the characteristic of the magnetic material to be designed, a representative dimension of the soft magnetic powder may be designed as the characteristic of the magnetic material.

(8) In the designing of the characteristic of the magnetic material to be designed in the method of designing low iron loss material in (7), in a case in which the magnetic material is a dust core made of soft magnetic powder, the representative dimension of the soft magnetic powder may be designed as the characteristic of the magnetic material based on a result of acquiring, for each representative dimension of the soft magnetic powder, the iron loss generated when the dust core is excited with a sinusoidal excitation waveform.

(9) An iron loss evaluation program according to an embodiment of the present disclosure causes a processor to execute the method of evaluating iron loss according to any one of (1) to (4).

(10) A low iron loss material design program according to an embodiment of the present disclosure causes a processor to execute the method of designing low iron loss material according to any one of (5) to (8).

(11) A low loss soft magnetic material according to an embodiment of the present disclosure has a characteristic designed by execution of the method of designing low iron loss material in (5) or (6).

(12) A low loss soft magnetic powder according to an embodiment of the present disclosure has a representative dimension designed by execution of the method of designing low iron loss material in (7) or (8).

(Advantageous Effect)

According to the method of evaluating iron loss and iron loss evaluation program of the present disclosure, the iron loss is directly predicted from the inverter conditions without measuring the iron loss when an iron core is excited by an inverter. According to the method of designing low iron loss material and low iron loss material design program, as well as the low loss soft magnetic material and low loss soft magnetic powder, of the present disclosure, iron loss is reduced when an iron core is excited by an inverter.

BRIEF DESCRIPTION OF THE DRAWINGS

In the accompanying drawings:

FIG. 1 is a diagram illustrating an example of the operation of a PWM inverter using a triangular wave comparison method;
FIG. 2 is a diagram illustrating an example of a frequency spectrum of an inverter excitation waveform;
FIG. 3 is a flowchart illustrating the procedure of a method for predicting iron loss according to a comparative example;
FIG. 4 is a flowchart illustrating an example of procedures of a method of evaluating iron loss according to an embodiment of the present disclosure;
FIG. 5 is a block diagram illustrating a configuration example of an information processing system according to an embodiment of the present disclosure;
FIG. 6 is a graph illustrating an example of the relationship between the carrier frequency and the frequency of a harmonic;
FIG. 7 is a graph illustrating an example of the relationship between the modulation factor and the frequency of a harmonic;
FIG. 8 is a graph illustrating an example of the relationship between the frequency of a modulated wave and the frequency of a harmonic;
FIG. 9 is a graph illustrating an example of the relationship between the carrier frequency and the magnetic flux density of a harmonic;
FIG. 10 is a graph illustrating an example of the relationship between the modulation factor and the magnetic flux density of a harmonic;
FIG. 11 is a graph illustrating an example of the relationship between the frequency of a modulated wave and the magnetic flux density of a harmonic;
FIG. 12 is a graph illustrating an example of the relationship between the measured value and the predicted value of the magnetic flux density of the harmonic;
FIG. 13 is an example of a graph used to determine $\alpha$ in the Steinmetz equation;
FIG. 14 is an example of a graph used to determine $\beta$ in the Steinmetz equation;
FIG. 15 is an example of a graph of the relationship between the value of $\alpha$ determined for a dust core using pure iron powder and the median size of the pure iron powder, expressed by simple regression;
FIG. 16 is an example of a graph of the relationship between the $\beta$ value determined for a dust core using pure iron powder and the median size of the pure iron powder, expressed by simple regression;
FIG. 17 is an example of a graph of the relationship between the y value determined for a dust core using pure iron powder and the median size of the pure iron powder, expressed by simple regression;
FIG. 18 is an example of a scatter plot illustrating the relationship between the measured values of iron loss and the values calculated based on the iron loss prediction formula in Example 1;
FIG. 19 is a graph illustrating an example of the relationship between the median size and the predicted value of iron loss in Example 2;
FIG. 20 is a graph illustrating an example of the relationship between the median size and the predicted value of iron loss in Example 3;
FIG. 21 is a graph in which the measured value of iron loss and value calculated based on the iron loss prediction formula in Example 4 are added to FIG. 18;
FIG. 22 is a diagram illustrating an example of eddy currents flowing in a dust core;
FIG. 23 is a diagram illustrating a configuration example of a dust core;
FIG. 24 is a scatter plot illustrating an example of the relationship between the eddy current loss coefficient of a sine wave and the cross-sectional area of a dust core;
FIG. 25 is a scatter plot illustrating an example of the relationship between the harmonic eddy current loss coefficient and the cross-sectional area of a dust core;
FIG. 26 is a scatter plot illustrating an example of the relationship between the actual measurement value and the predicted value of the sinusoidal eddy current iron loss in Example 5;
FIG. 27 is a scatter plot illustrating an example of the relationship between the measured values of the harmonic eddy current iron loss value and the predicted values without taking into account the harmonic eddy current loss coefficient in Example 6;
FIG. 28 is a scatter plot illustrating an example of the relationship between the measured values of the harmonic eddy current iron loss value and the predicted values taking into account the harmonic eddy current loss coefficient in Example 6; and

FIG. 29 is a scatter plot illustrating an example of the relationship between the actual measured values and the predicted values of iron loss in Example 7.

DETAILED DESCRIPTION

In recent years, power electronics, which utilize the switching function of semiconductors to efficiently control power, have undergone rapid development. By utilizing power electronics technology, conversion from AC to DC (converter), voltage conversion in DC (DC-DC converter), conversion from DC to AC (inverter), and AC frequency conversion (matrix converter) can be easily realized with relatively simple circuits. Furthermore, by increasing the switching frequency, the inductance or capacitance of passive elements required in the circuit is reduced. Reducing the inductance or capacitance of passive elements allows for device miniaturization. As there is a strong global demand for more efficient use of electricity and for smaller and lighter equipment in order to realize a carbon-neutral society, power electronics technology is now being applied not only to the power equipment, railways, and industrial fields where it has been applied preferentially until now, but also to electrical devices used in everyday life, such as automobiles and home appliances.

Inverters are one of the important circuits used in power electronic devices, and they not only convert DC to AC, but also efficiently control AC output. As familiar examples, inverters are used to control electric compressors in inverter air conditioners or induction motors for driving EVs (Electric Vehicles), and inverters make a significant contribution to energy conservation. For inverter output control, a PWM (Pulse Width Modulation) method is widely used, in which the pulse width of a constant voltage is modulated to control AC output. In a commonly used induction motor, an alternating current of several tens to several hundreds of hertz (Hz) is applied to rotate a rotor. On the other hand, a PWM inverter converts DC to AC for application to an induction motor to rotate the rotor. Therefore, in the PWM inverter, switching is performed at a frequency several times higher than the frequency of the AC current applied to the induction motor. An inverter using the PWM method is also called a PWM inverter.

In the present embodiment, the PWM inverter operates using a triangular wave comparison method. A triangular wave comparison type PWM inverter outputs a pulse waveform that is generated by comparing a target AC waveform with a triangular wave and switching a DC current. The time-averaged waveform of the output pulse waveform approximates the target AC waveform. The target AC waveform is also called a modulated wave. The triangular wave compared to the target AC waveform is also called a carrier wave. In other words, a PWM inverter using a triangular wave comparison method can convert a DC current into an AC waveform and output the AC waveform by switching the DC current based on the result of comparing the modulated wave with the carrier wave.

In the present embodiment, the PWM inverter generates the output pulse waveform by further utilizing a pulse waveform generated by comparing a waveform of the opposite phase of the modulated wave with a triangular wave. Specifically, the PWM inverter outputs a pulse waveform that is the difference between a pulse waveform obtained by inputting a modulated wave and a carrier wave into a comparator and switching the DC current based on the output of the comparator, and a pulse waveform obtained by inputting a waveform of the opposite phase of the modulated wave and the carrier wave into a comparator and switching the DC current based on the output of the comparator.

An example of the operation of a PWM inverter using a triangular wave comparison method will be described with reference to FIG. 1. The graph illustrated in FIG. 1 is a set of five graphs arranged on the same time scale. The horizontal axis of each graph represents time (t). The vertical axis of each graph represents signal strength.

The top graph illustrates the waveform of the carrier wave, as well as the waveform of the modulated wave and the waveform of the opposite phase of the modulated wave. The waveform of the carrier wave is represented as $e_c$. The frequency of the carrier wave, which is a triangular wave, is represented as $f_c$. The peak-to-peak value of the carrier wave waveform is represented as $E_c$.

The waveform of the modulated wave, which is a sine wave represented by a solid line, is represented as $e_{s1}$. Moreover, the waveform of the opposite phase of the modulated wave, which is a sine wave represented by a dashed line, is represented as $e_{s2}$. The peak-to-peak value of the modulated wave and the waveform of the opposite phase of the modulated wave is represented by $E_s$.

The ratio of $E_s$ to $E_c$ is also called the modulation factor and is denoted by m. In other words, the modulation factor (m) is the voltage amplitude ratio of the modulated wave to the carrier wave, and is calculated as $E_s/E_c$.

The PWM inverter can control the waveform of the AC current it outputs by controlling the modulation factor (m) and the carrier frequency ($f_c$). The modulation factor (m) and the carrier frequency ($f_c$) are also referred to as PWM parameters. The PWM parameters are included in the inverter conditions. The inverter conditions include not only PWM parameters but also parameters of the modulated wave. When the modulated wave is a sine wave, the parameters of the modulated wave are also referred to as sine wave conditions. That is, the inverter conditions include PWM parameters and sine wave conditions.

The second tier of the graph illustrates a pulse waveform generated based on a comparison between the carrier wave waveform and the modulated wave waveform. The pulse waveform generated based on a comparison between the carrier wave waveform and the modulated wave waveform is represented as $P_{s1}$. $P_{s1}$ is represented as a solid black rectangle in FIG. 1.

Specifically, $P_{s1}$ is generated so that it becomes 1 during the period when the intensity of the waveform of the modulated wave is equal to or greater than the intensity of the waveform of the carrier wave, and becomes 0 during the period when the intensity of the waveform of the modulated wave is less than the intensity of the waveform of the carrier wave. The 1 in the pulse waveform may be replaced with a HI signal. The 0 in the pulse waveform may be replaced with a LO signal.

The third tier of the graph illustrates a pulse waveform generated based on a comparison between the waveform of the carrier wave and the waveform of the opposite phase of the modulated wave. The pulse waveform generated based on a comparison between the waveform of the carrier wave and the waveform of the opposite phase of the modulated wave is represented as $P_{s2}$. $P_{s2}$ is illustrated as rectangles hatched with diagonal lines slanting downward to the right in FIG. 1.

Specifically, $P_{s2}$ is generated so that it becomes 1 during the period when the intensity of the waveform of the opposite phase of the modulated wave is equal to or greater than the intensity of the waveform of the carrier wave, and becomes 0 during the period when the intensity of the waveform of the modulated wave is less than the intensity of the waveform of the carrier wave.

The fourth tier of the graph illustrates a pulse waveform generated by subtracting the pulse waveform of $P_{s2}$ from the pulse waveform of $P_{s1}$. The pulse waveform yielded by subtracting the pulse waveform of $P_{s2}$ from the pulse waveform of $P_{s1}$ is denoted as $P_s$. $P_s$ is represented in FIG. 1 as rectangles with cross hatching.

Specifically, the pulse waveform of the circled dashed portion represented by P3 in the pulse waveform of $P_s$ is generated by subtracting the pulse waveform of the circled dashed portion represented by P2 in the pulse waveform of $P_{s2}$ from the pulse waveform of the circled dashed portion represented by P1 in the pulse waveform of $P_{s1}$. When $P_{s1}$ and $P_{s2}$ have the same value, the value of $P_s$ becomes zero. When $P_{s1}$ is 1 and $P_{s2}$ is 0, the value of $P_s$ becomes 1. When $P_{s1}$ is 0 and $P_{s2}$ is 1, the value of $P_s$ becomes -1. A pulse waveform in which $P_s$ is 1 is a positive pulse waveform. A pulse waveform in which $P_s$ is -1 is a negative pulse waveform. In other words, $P_s$ is a pulse waveform that combines a positive pulse waveform and a negative pulse waveform.

The fifth tier of the graph illustrates the time-averaged waveform of the $P_s$ pulse waveform. The time-averaged waveform of the $P_s$ pulse waveform is also referred to as the output waveform. The output waveform is an approximation of the modulated wave.

Here, it is assumed that a PWM inverter that is equipped with a SiC power semiconductor and outputs the output waveform exemplified in FIG. 1 is attached to the excitation power supply of a high-frequency iron loss measurement device. The excitation based on the output waveform of the PWM inverter is also called inverter excitation. An excitation waveform based on the output waveform exemplified in FIG. 1 was output using an excitation power supply equipped with a PWM inverter for inverter excitation of the magnetic material to be measured, thereby measuring the iron loss of the magnetic material. In the present embodiment, the frequency of the modulated wave is represented by $f_1$ and is set to 400 Hz to 1 kHz. The modulated wave frequency ($f_1$) is included in the sine wave conditions. For the modulated wave whose frequency range was set to 400 Hz to 1 kHz, the frequency ($f_c$) of the carrier wave was controlled in the range of 5 kHz to 100 kHz. The modulation factor (m) was controlled in the range of 0.4 to 1.25.

As described above, the excitation power supply of the high-frequency iron loss measurement device subjects the magnetic material to inverter excitation based on the output waveform of the PWM inverter. The waveform that is output when a magnetic material is subjected to inverter excitation is also called an inverter excitation waveform. The inverter excitation waveform may be considered to be the waveform produced by a PWM inverter. In the following, it is assumed that a PWM inverter generates the inverter excitation waveform.

Here, as one example, it is assumed that the PWM inverter generates an inverter excitation waveform based on a carrier wave having a carrier frequency ($f_c$) of 20 kHz and a modulation factor (m) of 0.4, and a modulation wave having a frequency ($f_1$) of 1 kHz. The inverter excitation waveform generated in this example is represented as a frequency spectrum as illustrated in FIG. 2 by applying a Fourier transform. In FIG. 2, the horizontal axis corresponds to frequency, designated as f. The unit of frequency is assumed to be kilohertz (kHz). The vertical axis corresponds to the intensity of each frequency component, represented as B. The intensity of each frequency component is assumed to be expressed in tesla (T).

The frequency spectrum of the inverter excitation waveform generated in this example mainly includes a modulated wave component having a frequency $f_1$ and a first-order harmonic component having a frequency $f_2$. In other words, the inverter excitation waveform is a waveform in which harmonics are superimposed on a modulated wave. The intensity of the modulated wave component is represented by $B_1$. The intensity of the modulated wave component is also called the excitation magnetic flux density. The excitation magnetic flux density ($B_1$) is included in the sine wave conditions. The intensity of the first harmonic component is represented as $B_2$.

In the frequency spectrum of FIG. 2, the frequency ($f_2$) of the first harmonic component is calculated as the average frequency of the most intense component other than the modulated wave component and the frequency of the component having an intensity of 50 % or more of that component. The frequency of the harmonic component contained in the frequency spectrum of FIG. 2 is 40 kHz. In other words, the frequency of the first harmonic component is twice the carrier frequency ($f_c$).

Harmonics include not only first-order components but also second-order and higher-order components. However, the second and higher harmonic components are negligibly small compared to the first harmonic component. Therefore, in the present embodiment, only the first harmonic component is considered. In the following description, the first harmonic component will simply be referred to as the harmonic component.

In general, data on the magnetic properties of iron core materials under sinusoidal excitation is made public, and motors are designed based on this data. However, as described above, the inverter excitation waveform using a PWM inverter is not a smooth sine wave, but contains harmonics resulting from the switching operation of semiconductors. Therefore, the iron loss occurring in the magnetic core during inverter excitation is the sum of the iron loss caused by the sinusoidal component and the iron loss caused by the harmonic component. As a result, in an inverter-driven motor using inverter excitation, the amount of heat generated becomes greater than that assumed at the time of design due to iron loss caused by the harmonic component. An increased amount of heat generated may necessitate cooling measures that were not anticipated during the design process. If the design is made with a margin of error, taking into consideration iron loss caused by harmonics, the size of the device using an inverter-driven motor will increase. In view of the strong demand for smaller devices and higher efficiency, it is necessary to estimate inverter iron loss accurately when designing devices.

When designing a motor and selecting a magnetic core material that is suitable for the motor's driving conditions, the iron loss under frequently used excitation conditions is often used as the evaluation index, rather than the iron loss under excitation conditions of the high magnetic flux density that corresponds to the motor's maximum output. The iron loss under frequently used excitation conditions may be, for example, $W_{10/400}$, which represents the iron loss when the excitation magnetic flux density ($B_1$) is 1.0 T and the frequency ($f_1$) is 400 Hz. However, the iron loss data that is generally disclosed, such as $W_{10/400}$, is data obtained with excitation by a sine wave, not with inverter excitation. Therefore, when designing a motor driven by an inverter, it is necessary to take into consideration the increase in iron loss caused by harmonics.

If a motor is connected to an inverter circuit that is actually used and the iron loss or amount of heat generated by the magnetic core is measured, the iron loss caused by inverter excitation can be directly evaluated. However, enormous cost and effort are required to directly evaluate the iron loss. In motor design, it is unrealistic to repeatedly fabricate prototype motors in order to directly evaluate iron loss starting at the stage of selecting the magnetic core material.

Alternatively, the iron loss of the test piece can be evaluated using an excitation power supply that simulates a PWM inverter. Moreover, the iron loss in inverter excitation can be evaluated by integrating the inverter excitation waveform synthesized based on the PWM parameters. However, this approach is difficult for reasons such as the requirement of a dedicated evaluation device and the complexity of the analysis method.

For these reasons, there is a need for a simple method for evaluating iron loss during inverter excitation.

Here, the PWM inverter can control the output by changing the modulation factor (m) from 0 to 1 while keeping the height of the pulse voltage constant. Furthermore, the PWM inverter can control the output by changing the carrier frequency ($f_c$). When evaluating iron loss, a specific inverter excitation waveform is applied to the magnetic material. Specifically, the modulated wave frequency ($f_1$) of the inverter excitation waveform applied to the magnetic material and the excitation magnetic flux density ($B_1$) are set as excitation conditions for evaluating iron loss.

The modulation factor (m) and carrier frequency ($f_c$) are set independently from the modulated wave frequency ($f_1$) of the inverter excitation waveform and the excitation magnetic flux density ($B_1$). In other words, there is no one-to-one correspondence between the excitation magnetic flux density ($B_1$) of the inverter excitation waveform, which is the excitation condition for evaluating iron loss, and the modulation factor (m). The modulation factor (m) may be set to a smaller value or a larger value relative to the excitation magnetic flux density ($B_1$). The value of the modulation factor (m) is set based on the output fluctuation range of the motor for which iron loss is to be evaluated or the design concept of the circuit.

When the modulation factor (m) is set to a large value (a value close to 1) with respect to the excitation magnetic flux density ($B_1$) of the inverter excitation waveform, the inverter iron loss tends to decrease. However, when the excitation magnetic flux density ($B_1$) is set to a high magnetic flux density region, an overmodulation state occurs in which the modulation factor (m) exceeds 1, and power control becomes unstable. Conversely, when the modulation factor (m) is set to a small value (a value close to 0), power control is stabilized in a case in which the excitation magnetic flux density ($B_1$) is set to a high magnetic flux density region, but inverter iron loss increases.

When the carrier frequency ($f_c$) is set to a high frequency, the intensity ($B_2$) of the harmonic component of the inverter excitation waveform becomes smaller, and an increase in iron loss caused by harmonics is suppressed. However, the heat generated by the switching elements increases, and the loss in the entire circuit also increases.

As described above, a PWM inverter can realize one excitation condition for evaluating iron loss by combining various different values of the modulation factor (m) or carrier frequency ($f_c$). The iron loss occurring during inverter excitation includes iron loss caused by harmonics determined according to the modulation factor (m) and carrier frequency ($f_c$). Therefore, even if the excitation conditions are the same, it is necessary to evaluate the iron loss taking into consideration the modulation factor (m) and carrier frequency ($f_c$) set by the PWM inverter to realize those excitation conditions.

Therefore, the method of evaluating iron loss according to the present disclosure can predict the iron loss caused by inverter excitation simply and with high accuracy based on the values of the modulation factor (m) and carrier frequency ($f_c$) that are set as control parameters of the PWM inverter for the modulated wave frequency ($f_1$) and the excitation magnetic flux density ($B_1$), which are the excitation conditions for which iron loss is to be evaluated. Furthermore, the method of designing low iron loss material according to the present disclosure can design the characteristics of a low iron loss material based on the predicted iron loss results, thereby providing a low iron loss material.

(Method of evaluating iron loss according to the present disclosure)

The method of evaluating iron loss according to the present disclosure uses a prediction formula that can calculate a predicted value of iron loss based on the values of the modulation factor (m) and carrier frequency ($f_c$), which are control parameters of the PWM inverter, in order to predict iron loss without measuring the excitation waveform when the magnetic material is subjected to inverter excitation.

The prediction formula for predicting iron loss based on the modulation factor (m) and carrier frequency ($f_c$) is constructed by the following procedure.

First, the magnetic flux density ($B_2$) and frequency ($f_2$) of the first harmonic that contributes significantly to iron loss are identified by performing Fourier analysis on an excitation waveform synthesized under a plurality of excitation conditions for inverter excitation by a PWM inverter. At this time, it is desirable to analyze an excitation waveform synthesized under three or more excitation conditions as the plurality of excitation conditions. The excitation conditions are specified by the modulation factor (m) and the carrier frequency ($f_c$).

Next, the relationship between the magnetic flux density ($B_2$) and frequency ($f_2$) of the first harmonic, which contributes significantly to iron loss, and the excitation conditions are substituted into the Steinmetz equation and rearranged to generate an iron loss prediction formula.

The iron loss prediction formula according to the present disclosure generated by the above procedure can calculate a predicted value of inverter iron loss directly from the excitation conditions of inverter excitation by a PWM inverter, without analysis of the excitation waveform.

As a comparative example, it is conceivable to perform extremely complicated calculations for analyzing the excitation waveform in order to predict the iron loss, or to measure the harmonic component for each excitation condition. The method of predicting iron loss according to the comparative example includes the steps illustrated in FIG. 3.

First, PWM parameters are set as inverter conditions for the PWM inverter (step S1). A sine wave condition may further be set as the inverter conditions. The magnetic material is subjected to inverter excitation by the waveform output by the PWM inverter under the set PWM parameters (step S2). The excitation waveform applied to the magnetic material is measured (step S3). The waveform data of the measured excitation waveform is integrated (step S4). Based on the result of integrating the excitation waveform, iron loss when the magnetic material is subjected to inverter excitation under the set PWM parameters is predicted (step S5).

As described above, the method according to the comparative example requires measurement of the excitation waveform and integration of the waveform data. In contrast, the method of evaluating iron loss according to the present disclosure is carried out only by the procedure illustrated in FIG. 4.

First, PWM parameters are set as inverter conditions for the PWM inverter (step S11). A sine wave condition may further be set as the inverter conditions. The set PWM parameters are input to a prediction formula prepared in advance (step S12). Based on the calculation result of the prediction formula, iron loss when the magnetic material is subjected to inverter excitation under the set PWM parameters is predicted (step S13).

As described above, the prediction formula according to the present disclosure can predict iron loss easily and with high accuracy without analyzing the excitation waveform.

Furthermore, the iron loss prediction formula according to the present disclosure can be expanded to a prediction formula that reflects the characteristics of the raw materials that make up the magnetic material, as will be described later. By reflecting the characteristics of the raw materials in the prediction formula, the prediction formula yields guidelines for designing magnetic materials that are suitable for inverter excitation and for which the iron loss occurring when the magnetic materials are subjected to inverter excitation is reduced.

<Example of hardware configuration for implementing the method of evaluating iron loss>

The method of evaluating iron loss according to the present disclosure may be realized by the information processing system 1 illustrated in FIG. 5. The information processing system 1 includes an information processing device 10, an excitation device 20, and a measurement device 30.

The excitation device 20 includes a PWM inverter and an excitation power supply. The PWM inverter may be realized with various circuits, such as a single-phase full-bridge circuit or a single-phase half-bridge circuit. The excitation power supply is configured to generate magnetic flux based on an output waveform from the PWM inverter and apply the magnetic flux to the magnetic material. The excitation power supply may be configured to pass a current corresponding to an output waveform from, for example, a PWM inverter through the excitation coil.

The measurement device 30 is configured to be able to measure the magnetic flux density when the magnetic material is subjected to inverter excitation. The measurement device 30 may include various magnetic sensors such as fluxgates or Hall elements.

The information processing device 10 includes a processor 12, a memory 14, and an interface 16.

The processor 12 may be configured to include, for example, a CPU (Central Processing Unit) or a GPU (Graphics Processing Unit) in order to control and manage various functions of the information processing device 10. The processor 12 may realize the functions of the information processing device 10 by reading and executing a program stored in the memory 14.

The memory 14 stores various types of information or data used by the information processing device 10. The memory 14 may, for example, store programs executed by the processor 12, or data used in the processing executed by the processor 12, the results of processing, and the like. The memory 14 may function as a working memory for the processor 12. The memory 14 may include, but is not limited to, a semiconductor memory, for example. The memory 14 may, for example, be configured as an internal memory of the processor 12 or may be configured as an electromagnetic recording medium such as a hard disk drive (HDD) that can be accessed by the processor 12. The memory 14 may be configured as a non-transitory readable medium. The memory 14 may be configured integrally with the processor 12 or may be configured separately from the processor 12.

The interface 16 may include a communication interface for communicating with other devices, such as the excitation device 20 or the measurement device 30, in a wired or wireless manner. The communication interface may be configured to communicate with other devices via a network. The interface 16 may be configured to include an input/output port for inputting and outputting data to and from other devices. The interface 16 may communicate based on wired communication standards or wireless communication standards. Wireless communication standards may, for example, include cellular phone communication standards such as 3G, 4G, and 5G. Wireless communication standards may, for example, include IEEE 802.11 and Bluetooth® (Bluetooth is a registered trademark in Japan, other countries, or both). The interface 16 may support one or more of these communication standards. The interface 16 is not limited to these examples and may communicate with other apparatuses and input or output data based on various standards.

The interface 16 may be configured to output information acquired from the processor 12. The interface 16 may notify the user of information by outputting visual information such as characters, figures, or images directly or via an external device. The interface 16 may include a display device, or may be connected to a display device in a wired or wireless manner. The display device may include a variety of displays, such as a liquid crystal display, for example. The interface 16 may notify the user of information by outputting auditory information such as voice, either directly or via an external device. The interface 16 may include an audio output device such as a speaker or may be connected to an audio output device in a wired or wireless manner. The interface 16 may not only notify the user of visual information or audio information, but may also notify the user of information by outputting information perceivable by the user directly, via an external device, or the like.

The interface 16 may include an input device that accepts input from the user. The input device may, for example, include a keyboard or physical keys, or may include pointing devices such as a touch panel, touch sensor, or mouse. The input device is not limited to these examples and may include a variety of other devices.

The processor 12 of the information processing device 10 may execute the method of evaluating iron loss. The method of evaluating iron loss may be realized as an iron loss evaluation program. The processor 12 may notify the user, via the interface 16, of the predicted value of iron loss calculated by executing the method of evaluating iron loss, may output the predicted value to an external device, or may store the predicted value in the memory 14.

<Example of constructing iron loss prediction formula>

An example of constructing a prediction formula will be specifically described below.

First, a magnetic material obtained by heat-treating a compact was prepared as a magnetic core for evaluating iron loss. The compact was prepared by applying a pressure of 980 MPa to iron powder and molding the iron powder into a ring shape.

The magnetic material was excited by, for example, applying the output waveform of the PWM inverter illustrated in FIG. 1 as an inverter excitation waveform to the magnetic material. The inverter excitation waveform is a waveform in which harmonics caused by semiconductor switching are superimposed on a modulated wave. The fact that the inverter excitation waveform is a waveform in which harmonics are superimposed on a modulated wave can be confirmed, for example, from the frequency spectrum graph illustrated in FIG. 2, in which the modulated wave frequency ($f_1$) component and the harmonic frequency ($f_2$) component are large.

<<Relationship between harmonic frequency ($f_2$) and inverter conditions>>

As described above, the harmonic frequency ($f_2$) component appearing in the frequency spectrum of FIG. 2 is 40 kHz. In other words, the harmonic frequency ($f_2$) is twice the carrier frequency ($f_c$). This relationship is confirmed by the relationship between the harmonic frequency ($f_2$) and the carrier frequency ($f_c$) when the magnetic material is excited by controlling the PWM inverter under a plurality of PWM parameters having different carrier frequencies ($f_c$), as illustrated in FIG. 6. In the graph of FIG. 6, the horizontal axis represents the carrier frequency ($f_c$). The vertical axis represents the harmonic frequency ($f_2$).

The harmonic frequency ($f_2$) depends on the carrier frequency ($f_c$) as described above, but does not depend on the modulation factor (m). This is confirmed by the relationship between the harmonic frequency ($f_2$) and the modulation factor (m) when the magnetic material is excited by controlling the PWM inverter under a plurality of PWM parameters with different modulation factors (m), as illustrated in FIG. 7. In the graph of FIG. 7, the horizontal axis represents the modulation factor (m). The vertical axis represents the harmonic frequency ($f_2$). The plotted points on the graph confirm that the harmonic frequency ($f_2$) does not change despite changes in the modulation factor (m).

Furthermore, the harmonic frequency ($f_2$) does not depend on the modulated wave frequency ($f_1$). This is confirmed by the relationship between the harmonic frequency ($f_2$) and the modulated wave frequency ($f_1$) when a magnetic material is excited with an excitation waveform having a different modulated wave frequency ($f_1$), as illustrated in FIG. 8. In the graph of FIG. 8, the horizontal axis represents the modulated wave frequency ($f_1$). The vertical axis represents the harmonic frequency ($f_2$). The plotted points on the graph confirm that the harmonic frequency ($f_2$) does not change despite changes in the modulated wave frequency ($f_1$).

Based on the relationships described above, the harmonic frequency ($f_2$) depends on the carrier frequency ($f_c$), is independent of the modulation factor (m) and the modulated wave frequency ($f_1$), and is expressed by expression (1) below.

[Math. 1]

$$f_2 = 2f_c \quad (1)$$

However, the proportionality constant used in expression (1) may differ depending on the type of PWM inverter. By generalizing the proportionality constant, the harmonic frequency ($f_2$) is expressed by expression (2) below.

[Math. 2]

$$f_2 = uf_c \quad (2)$$

The proportionality constant (u) used in expression (2) is set to 2 when the PWM inverter is a single-phase full-bridge circuit and is set to 1 when the PWM inverter is a single-phase half-bridge circuit. The proportionality constant is the ratio of the frequency of the harmonic of the excitation waveform to the carrier frequency. The proportionality constant may be set by directly checking the type of the PWM inverter. The proportionality constant may be determined by performing Fourier analysis on waveform data obtained by measuring, at least once, the excitation waveform when the magnetic material is subjected to inverter excitation by the PWM inverter, without directly checking the type of the PWM inverter. In other words, the proportionality constant may be determined based on waveform data obtained by measuring the excitation waveform generated by the PWM inverter.

As described above, it is clear that the harmonic frequency ($f_2$) is determined by the carrier frequency ($f_c$) among the inverter conditions.

<<Relationship between harmonic magnetic flux density ($B_2$) and inverter conditions>>

As illustrated in FIG. 9, the harmonic magnetic flux density ($B_2$) is proportional to the inverse of the carrier frequency ($f_c$). In other words, the harmonic magnetic flux density ($B_2$) is inversely proportional to the carrier frequency ($f_c$). In the graph of FIG. 9, the horizontal axis represents the carrier frequency ($f_c$). The vertical axis represents the harmonic magnetic flux density ($B_2$).

As illustrated in FIG. 10, the harmonic magnetic flux density ($B_2$) changes according to an exponential function that takes the modulation factor (m) as a variable. The exponent value ranged from -1.64 to -1.65. In the graph of FIG. 10, the horizontal axis represents the modulation factor (m). The vertical axis represents the harmonic magnetic flux density ($B_2$).

As illustrated in FIG. 11, the harmonic magnetic flux density ($B_2$) is proportional to the modulated wave frequency ($f_1$). In the graph of FIG. 11, the horizontal axis represents the modulated wave frequency ($f_1$). The vertical axis represents the harmonic magnetic flux density ($B_2$).

Based on the relationships described above, the harmonic magnetic flux density ($B_2$) is expressed by expression (3) below, using the carrier frequency ($f_c$), the modulation factor (m), and the modulated wave frequency ($f_1$).

$\gamma_1$ used in expression (3) is a proportionality constant.

[Math. 3]

$$B_2 = \gamma_1 \cdot f_c^{-1} \cdot m^{-1.64} \cdot f_1 \quad (3)$$

Here, the excitation power supply excites the magnetic material by applying a voltage to the excitation coil. The maximum value of the voltage applied by the excitation power supply is also called the maximum excitation voltage and is expressed in V. The relationship $V \propto B_1 \times f_1$ is established between the maximum excitation voltage (V), the modulated wave frequency ($f_1$), and the excitation magnetic flux density ($B_1$). Furthermore, if it is assumed that the carrier frequency ($f_c$) is changed so that the maximum excitation voltage (V) is constant, the relationship $V \propto B_2 \times f_c$ holds. From the above, it is assumed that the relationship $V \propto B_1 \times f_1 \propto B_2 \times f_c$ holds. Under this assumption, expression (4) below holds.

[Math. 4]

$$B_2 \propto \left(f_1/f_c\right) B_1 \quad (4)$$

It is also assumed that by applying expression (4) to expression (3), expression (5) below holds. $\gamma$ used in expression (5) is a proportionality constant.

[Math. 5]

$$B_2 \propto \gamma m^{-1.64} \left(f_1/f_c\right) B_1 \quad (5)$$

y included in expression (5) was determined by performing a regression analysis using expression (5) on the relationship between various inverter conditions and the harmonic magnetic flux density ($B_2$) obtained by Fourier analysis of the excitation waveform when the magnetic material was subjected to inverter excitation under each inverter condition. The harmonic magnetic flux density ($B_2$) was then predicted using expression (5) for which $\gamma$ was determined. The predicted value of the harmonic magnetic flux density ($B_2$) is represented with brackets as [$B_2$]. FIG. 12 illustrates an example of the relationship between the measured values and the predicted values of the harmonic magnetic flux density ($B_2$). In the graph of FIG. 12, the horizontal axis represents the measured value of the harmonic magnetic flux density ($B_2$). The vertical axis represents the predicted value ([$B_2$]) of the harmonic magnetic flux density. It is clear that the measured values of the harmonic magnetic flux density are proportional to the predicted values.

<<Calculating iron loss taking harmonics into account>>

The iron loss that occurs when a magnetic material is subjected to inverter excitation is expressed based on the Steinmetz equation illustrated as expression (6) below.

[Math. 6]

$$W_1 = W_{1.\mathrm{h}} + W_{1.\mathrm{e}} = \alpha \cdot f_1 \cdot B_1^{1.6} + \beta \cdot f_1^2 \cdot B_1^2 \quad (6)$$

In expression (6), $\alpha$ and $\beta$ are constants that are determined when the magnetic material is excited by a sinusoidal excitation waveform rather than inverter excitation. In other words, expression (6) represents the iron loss of sinusoidal excitation without taking into account harmonics during inverter excitation. The first term in expression (6) corresponds to the hysteresis loss and is represented as $W_{1.h}$. The second term corresponds to the eddy current loss and is represented as $W_{1.e}$.

It has been confirmed by measurements using a DC magnetizing measurement device that the hysteresis loss ($W_{1.h}$) is proportional to $f_1 \cdot B_1^{1.6}$, as illustrated in FIG. 13. Therefore, the constant $\alpha$ may be determined based on the measurement results illustrated in FIG. 13.

The constant $\beta$ for eddy current loss ($W_{1.e}$) may be determined by performing a regression analysis on the relationship between $f_1^2 \cdot B_1^2$ and $W_1 - \alpha \cdot f_1 \cdot B_1^{1.6}$ as illustrated in FIG. 14.

The iron loss taking into account inverter excitation is expressed as $W_{inv}$ and is also called inverter iron loss. The inverter iron loss ($W_{inv}$) is expressed by expression (7) below, which adds the harmonic iron loss ($W_{har}$), taking into account the influence of harmonics in the inverter excitation, to the sinusoidal excitation iron loss ($W_1$).

[Math. 7]

$$W_{\mathrm{inv}} = W_1 + W_{\mathrm{har}} \quad (7)$$

The harmonics that cause harmonic iron loss ($W_{har}$) are considered to be a sine wave having a frequency different from the frequency of the modulated wave. When the harmonics are considered to be a sine wave, it is assumed that the harmonic iron loss ($W_{har}$) can be separated into hysteresis loss and eddy current loss, similar to the iron loss ($W_1$) of sinusoidal excitation. Under this assumption, the harmonic iron loss ($W_{har}$) is expressed by expression (8) below.

[Math. 8]

$$W_{\mathrm{har}} = W_{\mathrm{har.h}} + W_{\mathrm{har.e}} = \alpha \cdot f_2 \cdot B_2^{1.6} + \beta \cdot f_2^2 \cdot B_2^2 \quad (8)$$

The inverter iron loss ($W_{inv}$) is calculated based on various inverter conditions that combine the modulated wave frequency ($f_1$), excitation magnetic flux density ($B_1$), carrier frequency ($f_c$), and modulation factor (m). The inverter iron loss ($W_{inv}$) is expressed as expression (9) below by substituting expressions (1), (5), (6), and (8) into the above-described expression (7).

[Math. 9]

$$W_{\mathrm{inv}} = \alpha \cdot (1 + p_{\mathrm{h}}) \cdot f_1 \cdot B_1^{1.6} + \beta \cdot (1 + p_{\mathrm{e}}) \cdot (f_1 \cdot B_1)^2 \quad (9)$$

In addition, in expression (9), the relationships $p_h = 2 \times \gamma^{1.6} \times m^{-2.62} \times (f_1/f_c)^{0.6}$ and $p_e = 4 \times \gamma^2 \times m^{-3.28}$ hold. The element of the harmonic iron loss caused by hysteresis loss is represented by $p_h$. The element of harmonic iron loss caused by eddy current loss is represented by $p_e$.

Predicting the inverter iron loss ($W_{inv}$) based on the modulation factor and the carrier frequency corresponds to calculating the inverter iron loss ($W_{inv}$) using the above-described expression (9). Inverter iron loss ($W_{inv}$) is iron loss that occurs when a magnetic material is excited by an excitation waveform generated by a PWM inverter controlled by inverter conditions including the modulation factor (m) and carrier frequency ($f_c$).

<<Summary>>

As described above, by calculating the inverter iron loss ($W_{inv}$) using expression (9), the iron loss is predicted taking into account the harmonic iron loss caused by harmonics during inverter excitation. In expression (9), the constants $\alpha$, $\beta$, and $\gamma$ are predetermined. Therefore, the predicted value of the inverter iron loss ($W_{inv}$) is calculated by substituting the inverter conditions, namely, the carrier frequency ($f_c$), the modulation factor (m), and the modulated wave frequency ($f_1$), as variables into expression (9). In other words, by substituting only the inverter conditions into expression (9) without substituting values based on the measured excitation waveform data, the inverter iron loss ($W_{inv}$) can be predicted. As a result, iron loss caused by inverter excitation can be predicted easily and with high accuracy without analyzing the excitation waveform.

(Method of designing low loss material according to present disclosure)

By using the above-described method of evaluating iron loss, expression (9) was constructed as a prediction formula that can predict iron loss during inverter excitation based only on inverter conditions. The constants $\alpha$ and $\beta$ included in the iron loss prediction formula change depending on the characteristics of the magnetic material to be excited. Therefore, by identifying the relationship between the constants and the characteristics of the magnetic material, information that serves as guidelines for designing the characteristics of the magnetic material so as to reduce the predicted value of iron loss can be obtained using the predicted value of iron loss. A method for optimizing materials based on prediction of iron loss using expression (9) will be described below. Optimization of a material involves not only determining a characteristic of the material as an optimal characteristic, but also determining an acceptable range for that optimal characteristic. The method of optimizing a material is also called a method of designing low loss material.

When the magnetic material is a dust core, the relationship between $\alpha$ and the median size of the iron powder used as the raw material for the dust core is expressed by expression (10) below.
[Math. 10]

$$\alpha = \frac{C_1}{D_{50}} + C_2 \quad (10)$$

The median size is the volume-based median of the particle size of the iron powder. That is, the median size is a particle size determined so that when iron powder is divided into two groups based on a certain particle size, the total volume of the larger particle size group is equal to the total volume of the smaller particle size group. The particle size of the iron powder is the diameter of the longest part of the iron powder. The median size is expressed as $D_{50}$. $C_1$ and $C_2$ are constants. FIG. 15 illustrates a graph of the relationship between the value of $\alpha$ determined for a dust core using pure iron powder and the median size ($D_{50}$) of the pure iron powder, expressed by simple regression. From this graph, $C_1$ is determined to be $1.19 \times 10^{-6}$ and $C_2$ is determined to be 0.0603.

Furthermore, when the magnetic material is a dust core, the relationship between $\beta$ and the median size of the iron powder used as the raw material for the dust core is expressed by expression (11) below.
[Math. 11]

$$\beta = C_3 {D_{50}}^{n} \quad (11)$$

In expression (11), n is set to a value between 1 and 2. $C_3$ is a constant. FIG. 16 illustrates a graph of the relationship between the value of $\beta$ determined for a dust core using pure iron powder and the median size ($D_{50}$) of the pure iron powder, expressed by simple regression. From this graph, $C_3$ is determined to be 7.65 and n is determined to be 1.46.

Regarding $\gamma$, which is a constant included in the iron loss prediction formula, FIG. 17 illustrates a graph of the relationship between the value of $\gamma$ determined for a dust core using pure iron powder and the median size ($D_{50}$) of the pure iron powder, expressed by simple regression. From this graph, it can be seen that the value of $\gamma$ is almost constant, regardless of the median size ($D_{50}$), and is determined to be the average value of 0.093.

As described above, the constants $\alpha$ and $\beta$ included in expression (9) can be replaced with an expression representing the characteristics of the magnetic material. By replacing $\alpha$ and $\beta$ in expression (9) with expressions (10) and (11), expression (12) below is derived as a prediction formula for inverter iron loss with the inverter conditions and the median size ($D_{50}$) of the precursor powder as variables.
[Math. 12]

$$W_{\mathrm{inv}} = \left(\frac{C_1}{D_{50}} + C_2\right)(1 + p_{\mathrm{h}}) f_1 B_1^{1.6} + (C_3 {D_{50}}^{n})(1 + p_{\mathrm{e}})(f_1 B_1)^2 \quad (12)$$

In the examples described above, $\alpha$ and $\beta$ were formulated based on the median size ($D_{50}$) of the particles that make up the dust core. Values representing the characteristics of a magnetic material may depend on the crystal grain size even when the magnetic material is a dust core. When the magnetic material is a stacked magnetic core, the value representing the characteristic of the magnetic material may also depend on the thickness of the stacked steel sheets. Therefore, the value representing the characteristics of a magnetic material is not limited to the median size ($D_{50}$). By expressing the characteristics of the magnetic material in terms of a representative dimension (D), expression (12) is thus generalized to expression (13).
[Math. 13]

$$W_{\mathrm{inv}} = \left(\frac{C_1}{D} + C_2\right)(1 + p_{\mathrm{h}}) f_1 B_1^{1.6} + (C_3 D^{n})(1 + p_{\mathrm{e}})(f_1 B_1)^2 \quad (13)$$

By using expression (13), guidelines can be obtained for design that allows optimization of the characteristics of the magnetic material to be used as a magnetic core, according to the inverter conditions for inverter excitation. The optimization of the magnetic material characteristics may be performed by calculating values that represent the material characteristics so that the predicted value of iron loss is minimized or reduced, as described below in the EXAMPLES section.

In other words, the iron loss that occurs when at least two types of magnetic materials having different characteristics are each excited with an excitation waveform generated by controlling a PWM inverter at a predetermined modulation factor and a predetermined carrier frequency may be predicted by executing the above-described method of evaluating iron loss. Then, based on the predicted value of iron loss, the characteristics of the magnetic material to be designed may be designed so as to reduce the iron loss occurring when the magnetic material to be designed is excited by the excitation waveform generated by controlling the PWM inverter at the predetermined modulation factor and the predetermined carrier frequency. Furthermore, as the characteristics of the magnetic material to be designed, a representative dimension of the magnetic material to be designed may be designed. When the magnetic material to be designed is a steel sheet, the representative dimension may be the sheet thickness or the like. When the magnetic material to be designed is a dust core, the representative dimension may be the crystal grain size of the dust core, or the like.

Furthermore, when the magnetic material is a dust core constituted by soft magnetic powder, the iron loss that occurs when the dust core is excited by a sinusoidal excitation waveform may be obtained for each representative dimension of the soft magnetic powder. Then, based on the iron loss for each representative dimension, the representative dimension of the soft magnetic powder may be designed as a characteristic of the magnetic material. Furthermore, in the case where the magnetic material is a steel sheet, the iron loss that occurs when the steel sheet is excited with a sinusoidal excitation waveform may be obtained for each representative dimension of the steel sheet. Then, based on the iron loss for each representative dimension, the representative dimension of the steel sheet may be designed as a characteristic of the magnetic material.

The processor 12 of the information processing device 10 may execute the method of designing low loss material. The method of designing low loss material may be realized as a low loss material design program. The processor 12 may notify the user, via the interface 16, of the material characteristics determined by executing the method of designing low loss material, may output the characteristics to an external device, or may store the characteristics in the memory 14.

A low loss soft magnetic material or low loss soft magnetic powder may be produced as a magnetic material having the designed characteristics by executing the above-described method of designing low loss material.

## EXAMPLES

Examples are now described.

### <Example 1>

A test dust core having an outer diameter of 38 mm, an inner diameter of 25 mm, and a height of 6 mm was produced by compressing and molding an insulated pure iron powder, having an average particle size of 96.8 μm, as the material powder at 980 MPa. For arbitrary PWM parameters, the measured value of iron loss of the test dust core, measured using an inverter iron loss measurement device, and the value calculated based on the iron loss prediction formula (9) by executing the method of evaluating iron loss according to the present disclosure, were obtained.

FIG. 18 illustrates a scatter plot representing the relationship between the measured values of iron loss and the values calculated based on the prediction formula (9). In the graph of FIG. 18, the horizontal axis represents the measured value of iron loss ($W_{inv}$). The vertical axis represents the value ($[W_{inv}]$) calculated based on the iron loss prediction formula (9).

The value calculated based on the iron loss prediction formula (9) corresponds to the predicted value of iron loss ($[W_{inv}]$). It was found that a roughly proportional relationship was observed between the measured value of iron loss ($W_{inv}$) and the predicted value of iron loss ($[W_{inv}]$). In other words, the measured value ($W_{inv}$) and the predicted value ($[W_{inv}]$) of iron loss are in good agreement.

### <Example 2>

Dust cores were prepared by compacting material powders having various median sizes. The predicted value of iron loss when the dust cores using the material powders of each median size were subjected to inverter excitation under inverter conditions of an excitation magnetic flux density ($B_1$) of 1.0 T, a modulated wave frequency ($f_1$) of 1 kHz, a carrier frequency ($f_c$) of 20 kHz, and a modulation factor (m) of 0.4 was calculated using the inverter iron loss prediction formula (9).

FIG. 19 illustrates the relationship between the median size and the predicted value of iron loss. In the graph of FIG. 19, the horizontal axis represents the median size ($D_{50}$). The vertical axis represents the predicted value of iron loss ($[W_{inv}]$). In the graph of FIG. 19, there exists a median size at which the predicted value of iron loss is minimum. The minimum predicted value of iron loss is represented by $[W_{inv}]_{min}$.

Here, it is assumed that the predicted value of iron loss is allowed to increase by about 3 % from its minimum value. The value that increases by 3 % from the minimum predicted value of the iron loss is represented on the vertical axis as $[W_{inv}]_{min} \times 1.03$. The range of the median size for keeping the predicted value of iron loss within the allowable range is the range of median sizes in which the predicted value of iron loss increases by no more than 3 % from its minimum value, and is represented by $D_{50_r}$. It can be seen from FIG. 19 that the allowable range of the median size is 46 μm to 113 μm. This range of median size may be referred to as a guideline for material design for forming a dust core.

<Example 3>

Dust cores were prepared by compacting material powders having various median sizes. The predicted value of iron loss when the dust cores using the material powders of each median size were subjected to inverter excitation under inverter conditions of an excitation magnetic flux density ($B_1$) of 1.0 T, a modulated wave frequency ($f_1$) of 1 kHz, a carrier frequency ($f_c$) of 20 kHz, and a modulation factor (m) of 0.4 or 0.6 was calculated using the inverter iron loss prediction formula (9). The predicted values of iron loss were calculated for each of the cases in which the modulation factor (m) was 0.4 and the modulation factor (m) was 0.6. In addition, the predicted value of the iron loss in the case of sinusoidal excitation instead of inverter excitation was calculated using the iron loss prediction formula (6) that does not include harmonic iron loss.

FIG. 20 illustrates the relationship between the median size and the predicted value of iron loss. In the graph of FIG. 20, the horizontal axis represents the median size ($D_{50}$). The vertical axis represents the predicted value of iron loss (Iron loss). The dashed dotted line graph represents the predicted value of iron loss when sinusoidal excitation is used. The solid line graph represents predicted values of iron loss when inverter excitation is performed with a modulation factor (m) of 0.4. The dashed line graph indicates predicted values of iron loss when inverter excitation is performed with a modulation factor (m) of 0.6.

The minimum predicted value of iron loss in the case of sinusoidal excitation is represented on the vertical axis as $IL_{min_sin}$. The minimum predicted value of iron loss in the case of inverter excitation with a modulation factor (m) of 0.4 is represented on the vertical axis as $IL_{min_m0.4}$. The minimum predicted value of iron loss in the case of inverter excitation with a modulation factor (m) of 0.6 is represented on the vertical axis as $IL_{min_m0.6}$. It can be seen that the median size ($D_{50}$) at which each minimum predicted value is obtained changes. This change in median size may be referred to as a guideline for material design for forming a dust core.

<Example 4>

The measured and predicted values of inverter iron loss were acquired for the case of subjecting the same test dust core as in Example 1 to inverter excitation under inverter conditions of an excitation magnetic flux density ($B_1$) of 0.5 T, a modulated wave frequency ($f_1$) of 400 Hz, a carrier frequency ($f_c$) of 20 kHz, and a modulation factor (m) of 0.4. FIG. 21 illustrates a scatter plot in which a point representing the relationship between the actual measured value and the predicted value of the inverter iron loss acquired under the aforementioned conditions is added as a solid triangle to the scatter plot of FIG. 18 illustrated in Example 1. Since the added point is located on a line representing the proportional relationship between the measured value ($W_{inv}$) and the predicted value ($[W_{inv}]$) of iron loss, it is clear that the predicted inverter iron loss value matches the measured value well even when the excitation magnetic flux density ($B_1$) is other than 1.0 T.

(Inverter iron loss prediction formula taking into account cross-sectional area of magnetic core)

The prediction formula described above was designed to be able to predict inverter iron loss under different excitation conditions, on the premise that the shape or magnetic properties of the magnetic core do not change. Here, the iron loss that occurs when inverter excitation is performed on a dust core may be affected by the cross-sectional area of the magnetic core. The cross-sectional area of a magnetic core is the area of a cross-section intersecting the magnetic flux that excites the magnetic core. The cross-sectional area of the magnetic core may be the area of a cross-section perpendicular to the magnetic path through the magnetic core. Below, it will be explained how the prediction formula for inverter iron loss can be expanded to a form that takes into account the cross-sectional area of the magnetic core, based on the results of an experiment confirming the relationship between the iron loss occurring in the magnetic core upon subjection to inverter excitation and the cross-sectional area of the magnetic core.

As a sample of a magnetic core to be evaluated for iron loss occurring upon subjection to inverter excitation, a magnetic material obtained by heat-treating a compact was prepared. The compact includes a plurality of iron powder particles 100, as illustrated in FIG. 22. Furthermore, a pressure of 980 MPa was applied to the compact to form a ring-shaped dust core 200 as illustrated in FIG. 23. The shape of a cross-section 210 intersecting the dust core 200 illustrated in FIG. 23 in the circumferential direction is rectangular. The shape of the cross-section 210 is not limited to a rectangle and may be various other shapes.

As illustrated in FIG. 22, the eddy current flowing in the dust core 200 includes an intra-particle eddy current I1 flowing in each iron powder particle 100 and an inter-particle eddy current I2 flowing between the iron powder particles 100. The loss due to the inter-particle eddy current I2 is significantly larger than the loss due to the intra-particle eddy current I1. Therefore, the iron loss due to eddy currents flowing in the dust core 200 depends on the area of the cross-section 210 of the dust core 200, that is, the cross-sectional area of the magnetic core. The cross-sectional area of the magnetic core is represented by S. As samples with different magnetic core cross-sectional areas S, a plurality of dust cores 200 with different heights H of the cross-section 210 were produced.

The harmonic eddy current loss $W_{har.e}$ is expressed by expression (14) below using a term including an element $p_e$ due to eddy current loss when the second term on the right-hand side of the above-described expression (9) is expanded. In expression (14), the relationships $p_e = 4\gamma^2 \cdot m^{-3.28}$ and $\gamma = 0.093$ hold.

[Math. 14]

$$W_{\mathrm{har.e}} = \beta \cdot p_{\mathrm{e}}(f_1 \cdot B_1)^2 \quad (14)$$

The sinusoidal eddy current loss $W_{1.e}$ of the dust core is calculated as the sum of the intra-particle eddy current loss $W_{1.e_intra}$ and the inter-particle eddy current loss $W_{1.e_inter}$, that is, by expression (15) below.

[Math. 15]

$$W_{\mathrm{1.e}} = W_{\mathrm{1.e_intra}} + W_{\mathrm{1.e_inter}} \quad (15)$$

The sinusoidal intra-particle eddy current loss $W_{1.e_intra}$ is calculated by expression (16) below. Here, $D_{50}$ is the median size of the iron powder particles 100, $C_4$ is a constant, $\rho_{intra}$ is the resistivity within the particle, and $D_p$ is the magnetic core density.

[Math. 16]

$$W_{\mathrm{1.e_intra}} = (\pi \cdot D_{50} \cdot B \cdot f)^2 / \left(\mathrm{C}_4 \cdot \rho_{\mathrm{intra}} \cdot D_{\mathrm{p}}\right) \quad (16)$$

The sinusoidal inter-particle eddy current loss $W_{1.e_inter}$ is calculated by expression (17) below. Here, $C_5$ is a constant, and $\rho_{inter}$ is the resistivity of the entire magnetic core.

[Math. 17]

$$W_{\mathrm{1.e_inter}} = \left(\pi \cdot \sqrt{S} \cdot B \cdot f\right)^2 / \left(\mathrm{C}_5 \cdot \rho_{\mathrm{inter}} \cdot D_{\mathrm{p}}\right) \quad (17)$$

Here, it is assumed that the square of the median size $D_{50}$ of the iron powder particles 100 is proportional to the cross-sectional area of the iron powder particles 100. By applying the above-described expression (15), (16), and (17) to the relational expression $W_{1.e} = \beta(f_1 \cdot B_1)^2$ included in the above-described expression (6) and rearranging, the eddy current loss coefficient $\beta$ is derived as illustrated in expression (18) below.

[Math. 18]

$$\beta = {D_{50}}^2 / (\mathrm{C}_4 \cdot \rho_{\mathrm{intra}}) + S / (\mathrm{C}_5 \cdot \rho_{\mathrm{inter}}) \quad (18)$$

In order to verify the possibility that the inter-particle eddy current loss changes with the change in the cross-sectional area, the eddy current loss coefficient $\beta$ is expressed as a linear function of S as illustrated in expression (19) below on the assumption that $D_{50}$, $\rho_{intra}$, and $\rho_{inter}$ in expression (18) are constant.

[Math. 19]

$$\beta = aS + b \quad (19)$$

Here, in order to determine the relationship between the cross-sectional area S of the dust core and the eddy current loss coefficient β, the eddy current loss in the dust cores produced with four different heights was measured. The dust cores used to measure the eddy current loss were four types of test dust cores obtained by compressing a compact made of an insulated pure iron powder, having a median size of 87.7 μm, at 980 MPa. The four types of test dust cores all have the same outer diameter of 38 mm and inner diameter of 25 mm. The heights of the four types of test dust cores are 4 mm, 6 mm, 10 mm, and 18 mm, respectively. The cross-sectional areas of the four types of test dust cores are 25 $mm^2$, 38 $mm^2$, 64 $mm^2$, and 116 $mm^2$, respectively.

The eddy current loss coefficient β is calculated from the actual measured value of eddy current loss when sinusoidal excitation is performed on the dust core. FIG. 24 illustrates a scatter plot of an example of the relationship between the values of β calculated from the measured eddy current loss values when sinusoidal excitation is performed on the dust cores produced with four different heights, and the cross-sectional areas S of the dust cores. By performing a simple regression analysis on the four points plotted in the scatter plot, the coefficients when the relationship with the cross-sectional area S of the dust core was approximated by expression (19) were calculated as a = 0.325 and b = $7.25 \times 10^{-6}$.

Here, the eddy current loss coefficient β expressed by expression (19) was applied to expression (14) to predict the harmonic eddy current loss $W_{har.e}$. When the predicted value of the harmonic eddy current loss $[W_{har.e}]$ was compared with the measured value of the eddy current loss of the harmonic loss $W_{har}$ obtained from the inverter iron loss measurement, it was found that the prediction accuracy of the harmonic eddy current loss was low. One of the reasons for the low prediction accuracy of harmonic eddy current loss is the eddy current loss coefficient β. Specifically, when it is assumed that the eddy current loss coefficient β depends on the material but does not depend on the excitation conditions, β is expressed as in the above-described expression (19). In other words, the fact that the same value is applied as the eddy current loss coefficient β in both the case of sinusoidal excitation and the case of inverter excitation is one of the reasons for the low prediction accuracy of the harmonic eddy current loss.

Conversely, the fact that the prediction accuracy of the harmonic eddy current loss was low suggests that in the case of inverter excitation, the eddy current loss due to the sinusoidal component and the eddy current loss due to the harmonic component need to be calculated separately. Therefore, the eddy current loss coefficient β may be divided into a sinusoidal eddy current loss coefficient $\beta_{sin}$ and a harmonic eddy current loss coefficient $\beta_{har}$. The sinusoidal eddy current loss coefficient $\beta_{sin}$ is assumed to be the same as β expressed by the above-described expression (19).

In the inverter iron loss prediction formula taking into account the cross-sectional area S of the dust core, the inverter iron loss prediction formula to which the harmonic eddy current loss coefficient $\beta_{har}$ is applied is expressed as expression (20) below.

[Math. 20]

$$[W_{\mathrm{inv}}] = \alpha(1 + p_{\mathrm{h}})f_1 \cdot B_1^{1.6} + \{\beta_{\mathrm{sin}} + \beta_{\mathrm{har}}p_{\mathrm{e}}\}(f_1 B_1)^2 \quad (20)$$

The harmonic eddy current loss coefficient $\beta_{har}$ is expressed as expression (21) below in accordance with the sinusoidal eddy current loss coefficient $\beta_{sin}$, i.e., the form of β expressed in expression (19).

[Math. 21]

$$\beta_{\mathrm{har}} = a'S + b' \quad (21)$$

The harmonic eddy current loss coefficient $\beta_{har}$ is calculated from the measured value of eddy current loss when the dust core is subjected to inverter excitation. FIG. 25 illustrates a scatter plot of an example of the relationship between the values of $\beta_{har}$ calculated from the measured eddy current loss values when inverter excitation is performed on the dust cores produced with four different heights, and the cross-sectional areas S of the dust cores. By performing a simple regression analysis on the four points plotted in the scatter plot, the coefficients a' = 0.860 and b' = $2.02 \times 10^{-6}$ were calculated when the relationship with the cross-sectional area S of the dust core was approximated by expression (21).

Here, by applying β in expression (19) to $\beta_{sin}$ included in expression (20) and applying $\beta_{har}$ in expression (21) to $\beta_{har}$, a prediction formula for inverter iron loss with the inverter conditions and the cross-sectional area S of the dust core as parameters is expressed as expression (22) below.

[Math. 22]

$$[W_{\mathrm{inv}}] = \alpha(1 + p_{\mathrm{h}})f_1 \cdot B_1^{1.6} + \{(aS + b) + (a'S + b')p_{\mathrm{e}}\}(f_1 B_1)^2 \quad (22)$$

The correlation between the harmonic eddy current loss coefficient and the cross-sectional area of the dust core is reflected in the prediction formula, as described above. Hence, the phenomenon whereby the measured eddy current loss value increases at an accelerated rate as the cross-sectional area of the dust core increases is reflected in the prediction of the harmonic eddy current loss. As a result, the prediction accuracy of high frequency eddy current loss is improved.

<Example 5>

As a magnetic core sample, a test dust core was produced by compressing a compact made of an insulated pure iron powder, having a median size of 87.7 μm, at 980 MPa. The test dust core was ring-shaped with an outer diameter of 38 mm, an inner diameter of 25 mm, and a height of 10 mm. The area of a cross-sectional intersecting the test dust core in the circumferential direction was 64 $mm^2$.

The actual eddy current iron loss of the test dust core under arbitrary sinusoidal excitation conditions was measured using a sinusoidal iron loss measurement device. In addition, the predicted value of the eddy current iron loss of the test dust core under arbitrary sinusoidal excitation conditions was calculated using the above-described sinusoidal eddy current loss prediction formula (15) and expression (19).

FIG. 26 illustrates a scatter plot of an example of the relationship between the measured value $W_{1.e}$ and the predicted value $[W_{1.e}]$ of the sinusoidal eddy current iron loss. In the graph of FIG. 26, the horizontal axis represents the measured value $W_{1.e}$ of the sinusoidal eddy current iron loss. The vertical axis represents the predicted value $[W_{1.e}]$ of the sinusoidal eddy current iron loss. A roughly proportional relationship is observed between the measured value $W_{1.e}$ and the predicted value $[W_{1.e}]$ of the sinusoidal eddy current iron loss. In addition, the measured value $W_{1.e}$ and the predicted value $[W_{1.e}]$ of the sinusoidal eddy current iron loss are in good agreement.

<Example 6>

As a magnetic core sample, a test dust core similar to that in Example 5 was produced.

The actual eddy current iron loss of the test dust core under arbitrary PWM parameters was measured using an inverter iron loss measurement device. In addition, the predicted value of the eddy current iron loss of the test dust core under arbitrary PWM parameters was calculated using the above-described harmonic eddy current loss prediction formula (14) and expression (19).

FIG. 27 illustrates a scatter plot of an example of the relationship between the measured value $W_{har.e}$ and the predicted value $[W_{har.e}]$ of the harmonic eddy current iron loss. In the graph of FIG. 27, the horizontal axis represents the measured value $W_{har.e}$ of the harmonic eddy current iron loss. The vertical axis represents the predicted value $[W_{har.e}]$ of the harmonic eddy current iron loss. A roughly proportional relationship is observed between the measured value $W_{har.e}$ and the predicted value $[W_{har.e}]$ of the harmonic eddy current iron loss. However, the predicted value $[W_{har.e}]$ is about 1/2 of the measured value $W_{har.e}$. In other words, the prediction accuracy of the harmonic eddy current iron loss is low when $\beta$ expressed by expression (19) is used.

On the other hand, the predicted value of the eddy current iron loss of the test dust core at arbitrary PWM parameters was calculated using the second term on the right-hand side of expression (22), to which $\beta_{har}$ represented by the above-described expression (21) is applied in addition to $\beta$ represented by expression (19), i.e., $\beta_{sin}$. FIG. 28 illustrates a scatter plot of an example of the relationship between the measured value $W_{har.e}$ and the predicted value $[W_{har.e}]$ of the harmonic eddy current iron loss. In the graph of FIG. 28, the horizontal axis represents the measured value $W_{har.e}$ of the harmonic eddy current iron loss. The vertical axis represents the predicted value $[W_{har.e}]$ of the harmonic eddy current iron loss. In FIG. 28, the predicted value $[W_{har.e}]$ is about 1.07 times the value of the measured value $W_{har.e}$. As compared with the above-described prediction using only $\beta$, the prediction accuracy of the harmonic eddy current iron loss is higher when prediction is made using $\beta_{sin}$ and $\beta_{har}$. In other words, by applying $\beta_{har}$ to the prediction formula, the prediction accuracy is improved.

<Example 7>

As a magnetic core sample, a test dust core similar to that in Example 5 was produced.

The actual iron loss, which is a sum of the eddy current loss and the hysteresis loss, of the test dust core at arbitrary PWM parameters was actually measured using an inverter iron loss measurement device. In addition, the predicted value of the iron loss of the test dust core at arbitrary PWM parameters was calculated using the above-described inverter iron loss prediction formula (22).

FIG. 29 illustrates a scatter plot of an example of the relationship between the measured value $W_{inv}$ and the predicted value $[W_{inv}]$ of the inverter iron loss. In the graph of FIG. 29, the horizontal axis represents the measured value $W_{inv}$ of inverter iron loss. The vertical axis represents the predicted value $[W_{inv}]$ of inverter iron loss. An approximately proportional relationship is observed between the measured value $W_{inv}$ and the predicted value $[W_{inv}]$ of the inverter iron loss value. In addition, the measured value $W_{inv}$ and the predicted value $[W_{inv}]$ of the inverter iron loss are in good agreement.

(Summary of inverter iron loss prediction formula taking into account cross-sectional area of magnetic core)

As described above, by predicting the inverter iron loss when the dust core is subjected to inverter excitation based additionally on the cross-sectional area of the dust core, the prediction accuracy of the inverter iron loss when the cross-sectional area of the dust core changes is improved. For example, a prediction formula created based on measured values of inverter iron loss in a dust core sample with a small cross-sectional area can be used to predict, with high accuracy, the inverter iron loss in a large cross-sectional area dust core used in a motor.

The above-described inverter iron loss prediction formula is expressed using a formula that represents $\beta_{sin}$ and $\beta_{har}$ when the median size of the iron powder, which is the material of the dust core, is a specific value. In other words, a prediction formula for inverter iron loss may be created for each median size of iron powder.

While embodiments of the present disclosure have been described with reference to the drawings and examples, it should be noted that various modifications and revisions may be implemented by those skilled in the art based on the present disclosure. Accordingly, such modifications and revisions are included within the scope of the present disclosure. For example, functions or the like included in each component, each step, or the like can be rearranged without logical inconsistency, and a plurality of components, steps, or the like can be combined into one or divided. Embodiments according to the present disclosure can also be realized as a program executed by a processor included in an apparatus or as a storage medium having the program recorded thereon. Such embodiments are also to be understood as included in the scope of the present disclosure.

The mathematical expressions used in the above-described embodiments can be modified in various ways.

For example, the exponent "1.6" used in the first term (the hysteresis loss term) of the Steinmetz equation may be replaced with another value.

Furthermore, $p_h$ and $p_e$ included in expression (9) and the like may be formulated as $p_h = u \times (\gamma \times m^s)^{1.6} \times (f_1/f_c)^{0.6}$ and $p_e = (u \times \gamma \times m^s)^2$, using u, which is a coefficient determined by the type of inverter circuit, or letting the ratio of the exponent of m to the exponent of $\gamma$ be s. The coefficient u, which is determined by the type of inverter circuit, is the ratio between the frequency ($f_2$) of the first harmonic contained in the inverter excitation waveform and the carrier frequency ($f_c$). The values $\gamma$ and s express the relationship between the magnetic flux density ($B_2$) of the first harmonic and the excitation magnetic flux density ($B_1$) by the expression $(B_2 \times f_c)/(B_1 \times f_1) = y \times m^s$ and are values determined based on the type of the target inverter circuit, or values calculated based on a prior waveform analysis using a circuit of the same type.

REFERENCE SIGNS LIST

1 Information processing system
10 Information processing device (12: Processor, 14: Memory, 16: Interface)
20 Excitation device
30 Measurement device

## Claims

1. A method of evaluating iron loss comprising predicting, based on a modulation factor and a carrier frequency, iron loss that occurs when a magnetic material is excited by an excitation waveform generated by a PWM inverter that is controlled using PWM parameters including the modulation factor and the carrier frequency.

2. The method of evaluating iron loss according to claim 1, wherein in the predicting of the iron loss, the iron loss is predicted based on a ratio of a frequency of a harmonic of the excitation waveform to the carrier frequency.

3. The method of evaluating iron loss according to claim 2, wherein in the predicting of the iron loss, a ratio of a frequency of a harmonic of the excitation waveform to the carrier frequency is determined based on waveform data obtained by measuring the excitation waveform generated by the PWM inverter.

4. The method of evaluating iron loss according to any one of claims 1 to 3, wherein when the magnetic material is a dust core, the iron loss is predicted further based on a cross-sectional area of the dust core that intersects with a magnetic flux that excites the dust core.

5. A method of designing low iron loss material, comprising designing a characteristic of a magnetic material to be designed so as to reduce iron loss occurring when the magnetic material to be designed is excited by an excitation waveform generated by controlling a PWM inverter at a predetermined modulation factor and a predetermined carrier frequency, based on a result of executing the method of evaluating iron loss according to any one of claims 1 to 4 to predict iron loss occurring when each of at least two types of magnetic materials having different characteristics is excited by an excitation waveform generated by controlling the PWM inverter at the predetermined modulation factor and the predetermined carrier frequency.

**6.** The method of designing low iron loss material according to claim 5, wherein in the designing of the characteristic of the magnetic material to be designed, a representative dimension of the magnetic material to be designed may be designed as the characteristic of the magnetic material to be designed based on a result of acquiring, for each representative dimension of the magnetic material to be designed, the iron loss generated when the magnetic material to be designed is excited by a sinusoidal excitation waveform.

**7.** The method of designing low iron loss material according to claim 5, wherein the magnetic material to be designed is a dust core made of soft magnetic powder, and
in the designing of the characteristic of the magnetic material to be designed, a representative dimension of the soft magnetic powder is designed as the characteristic of the magnetic material.

**8.** The method of designing low iron loss material according to claim 7, wherein in the designing of the characteristic of the magnetic material to be designed, the representative dimension of the soft magnetic powder is designed as the characteristic of the magnetic material based on a result of acquiring, for each representative dimension of the soft magnetic powder, the iron loss generated when the dust core is excited with a sinusoidal excitation waveform.

**9.** An iron loss evaluation program configured to cause a processor to execute the method of evaluating iron loss according to any one of claims 1 to 4.

**10.** A low iron loss material design program configured to cause a processor to execute the method of designing low iron loss material according to any one of claims 5 to 8.

**11.** A low loss soft magnetic material having a characteristic designed by execution of the method of designing low iron loss material according to claim 5 or 6.

**12.** A low loss soft magnetic powder having a representative dimension designed by execution of the method of designing low iron loss material according to claim 7 or 8.

FIG. 1

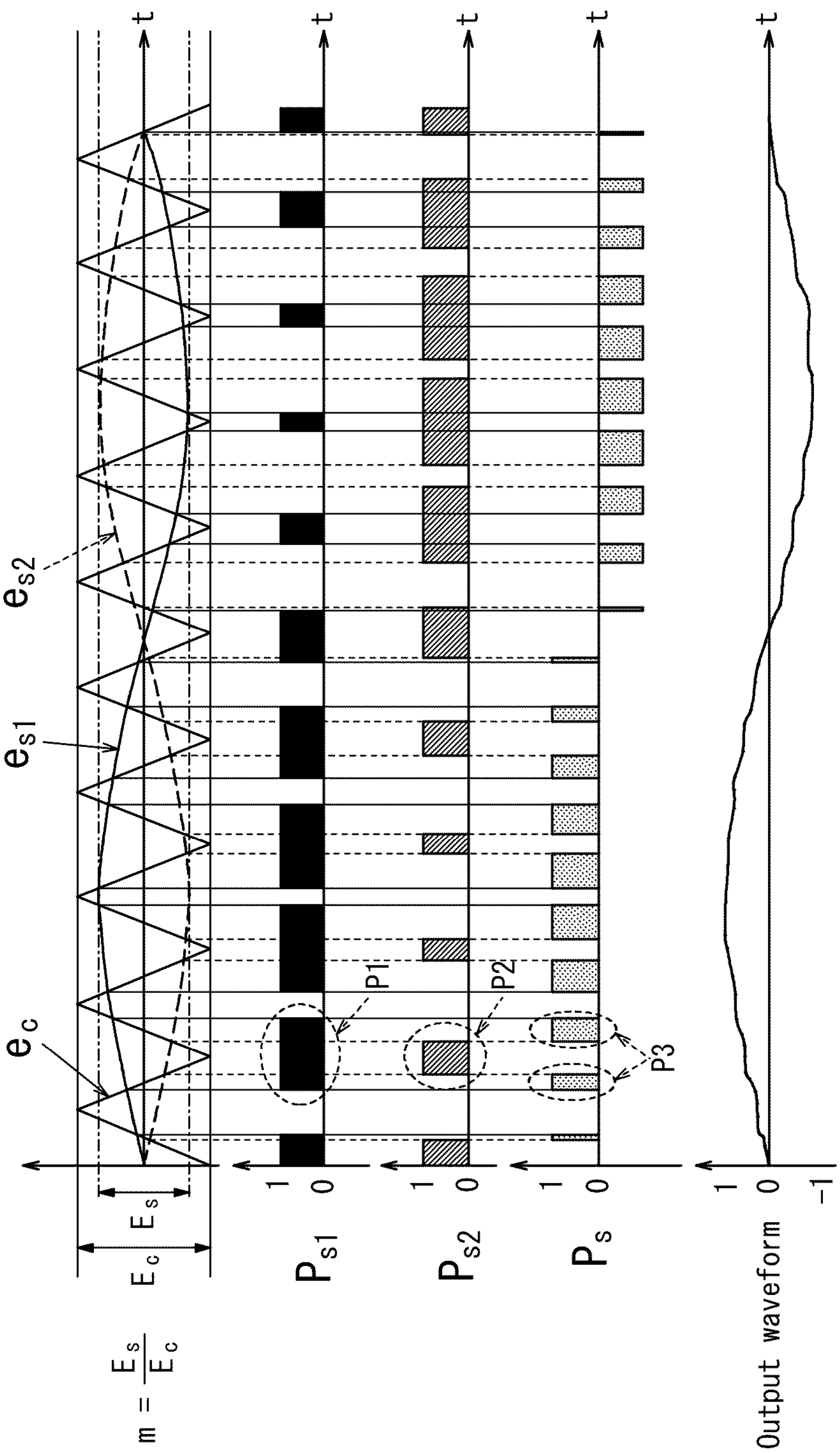

$e_c$
$e_{s1}$
$e_{s2}$
$m = \frac{E_s}{E_c}$
$E_c$
$E_s$
$P_{s1}$
$P_{s2}$
$P_s$
P1
P2
P3
Output waveform
1
0
-1
t

FIG. 2

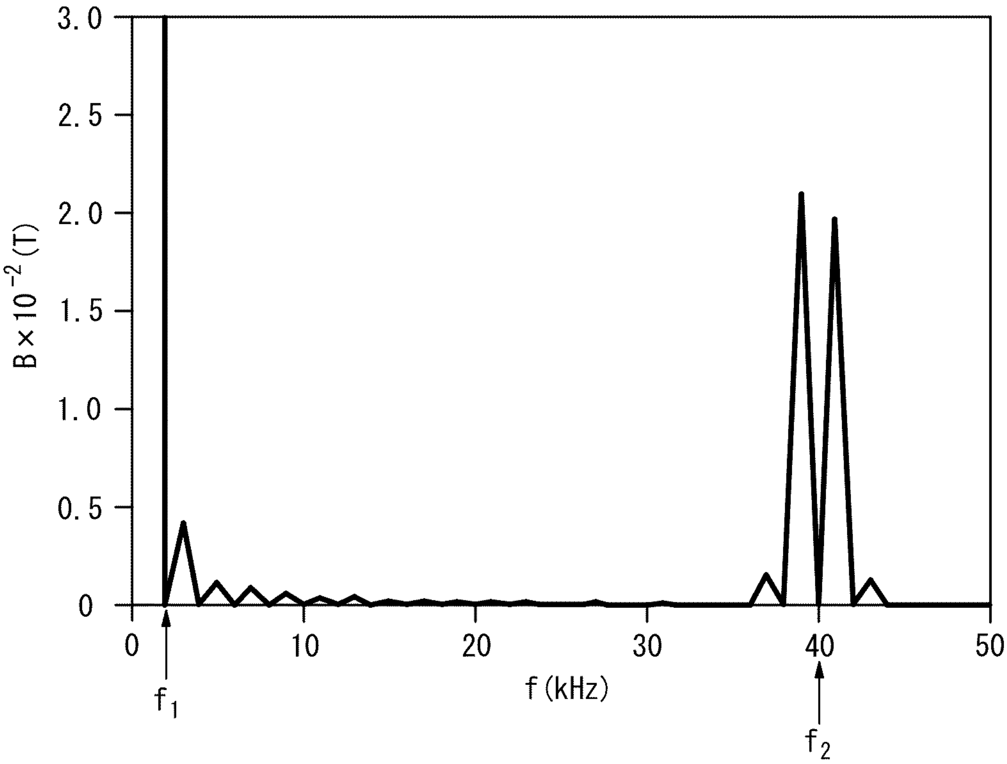

FIG. 3

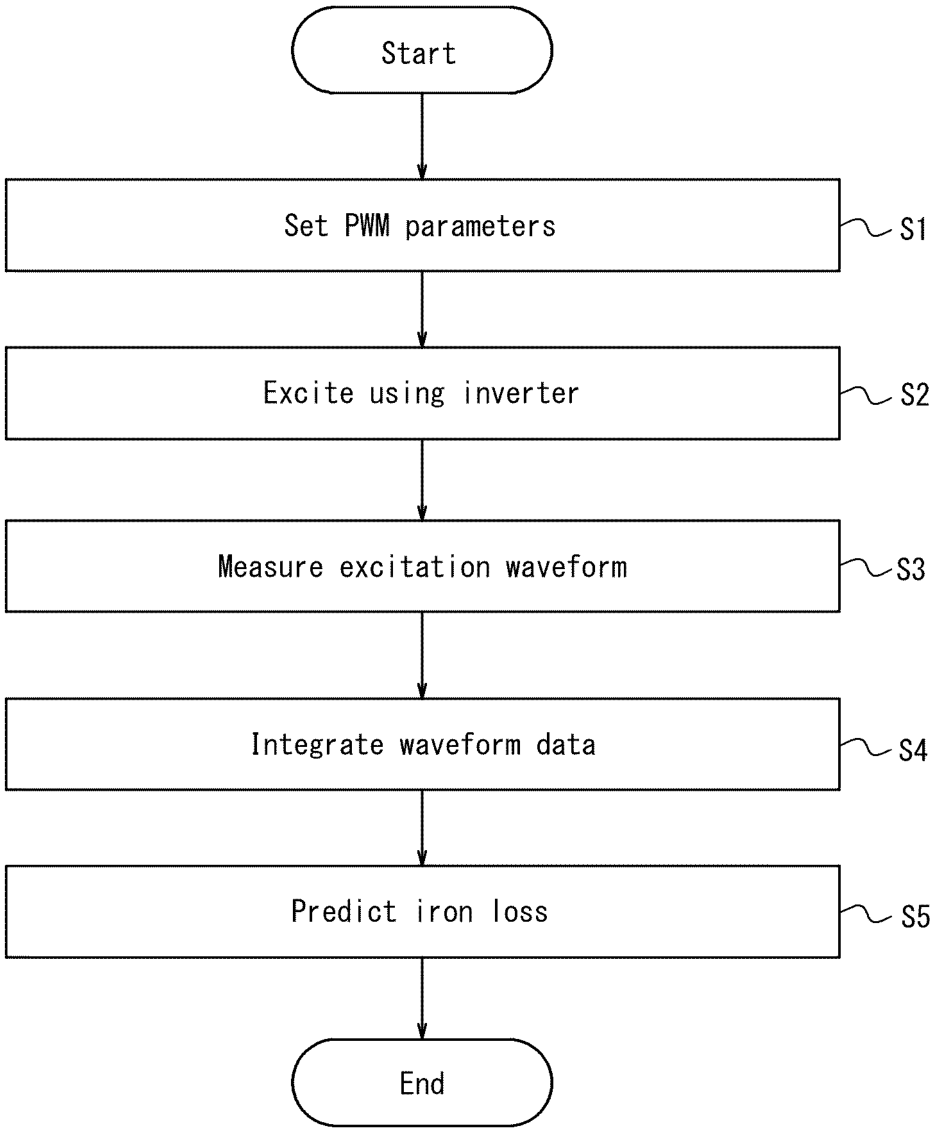
Start
Set PWM parameters
S1
Excite using inverter
S2
Measure excitation waveform
S3
Integrate waveform data
S4
Predict iron loss
S5
End

FIG. 4

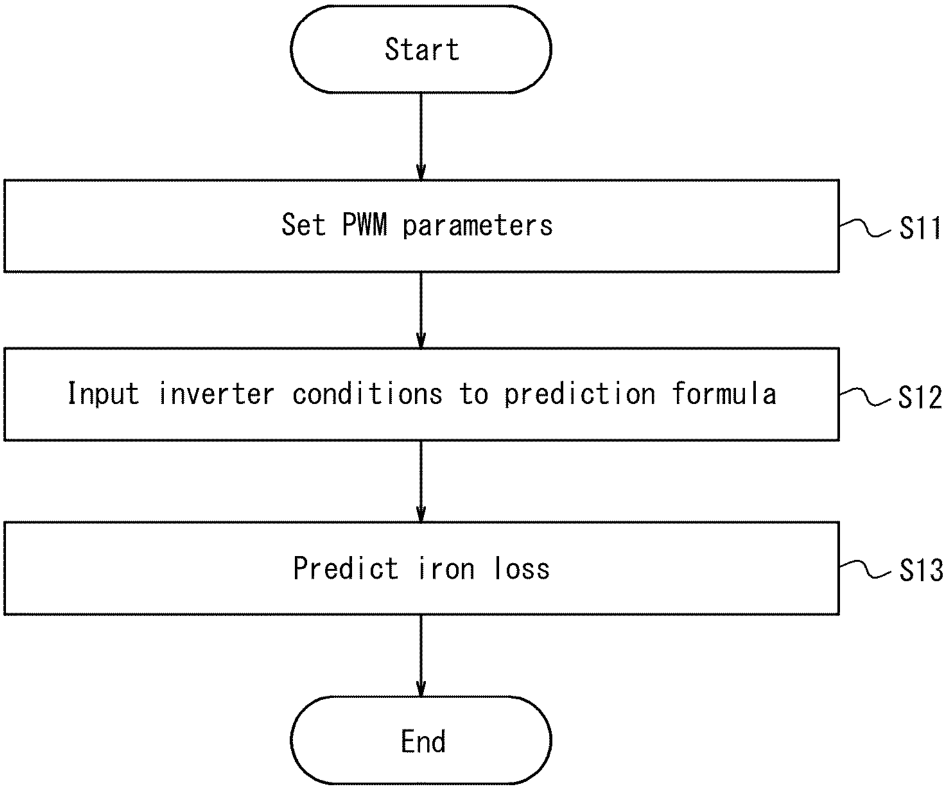
Start
Set PWM parameters
S11
Input inverter conditions to prediction formula
S12
Predict iron loss
S13
End

FIG. 5

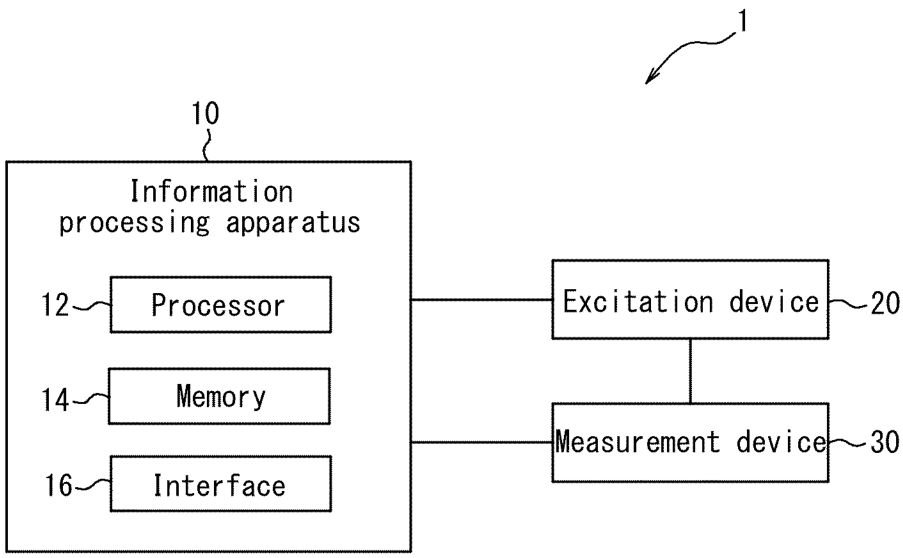
1
10
Information processing apparatus
12
Processor
14
Memory
16
Interface
Excitation device
20
Measurement device
30

FIG. 6

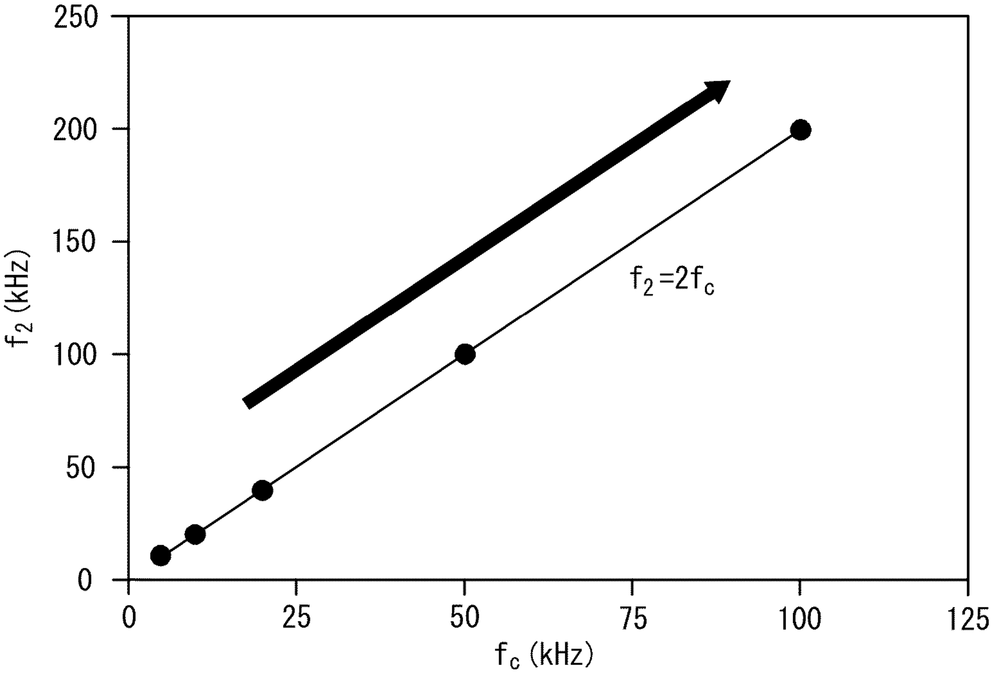
250
200
150
100
50
0
f2 (kHz)
f2=2fc
0
25
50
75
100
125
fc (kHz)

FIG. 7

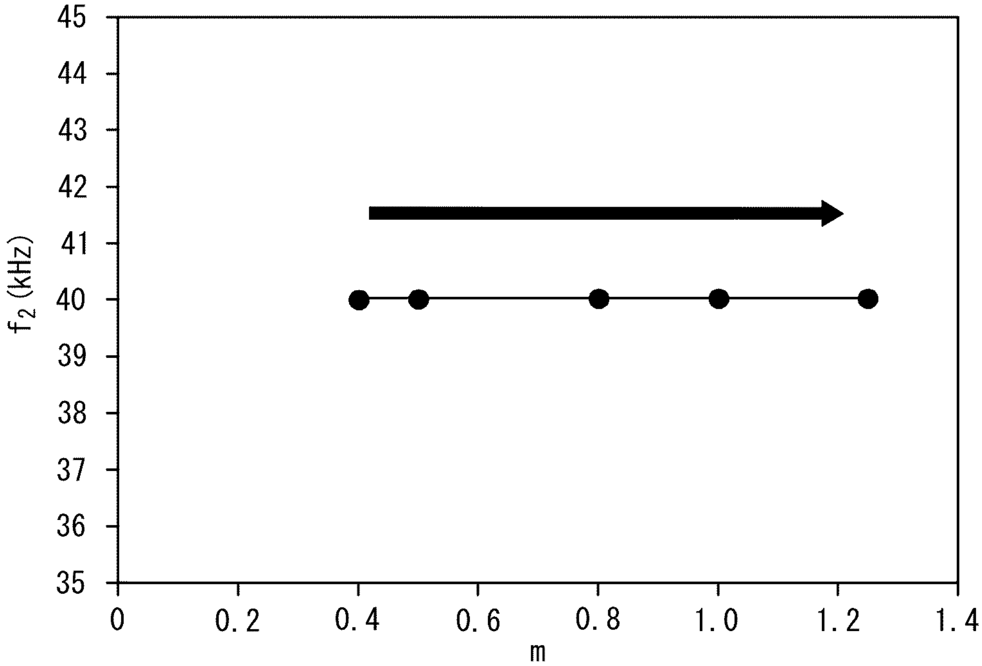
45
44
43
42
41
40
39
38
37
36
35
f2 (kHz)
0
0.2
0.4
0.6
0.8
1.0
1.2
1.4
m

FIG. 8

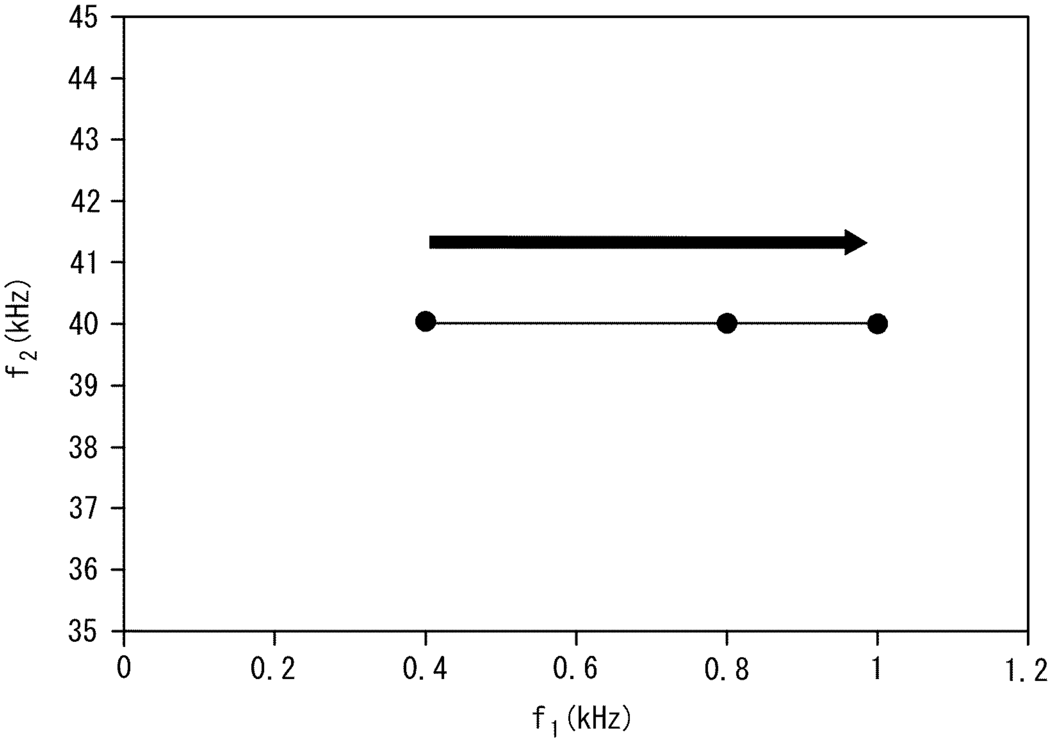
45
44
43
42
41
40
39
38
37
36
35
f 2 (kHz)
0
0. 2
0. 4
0. 6
0. 8
1
1. 2
f 1 (kHz)

FIG. 9

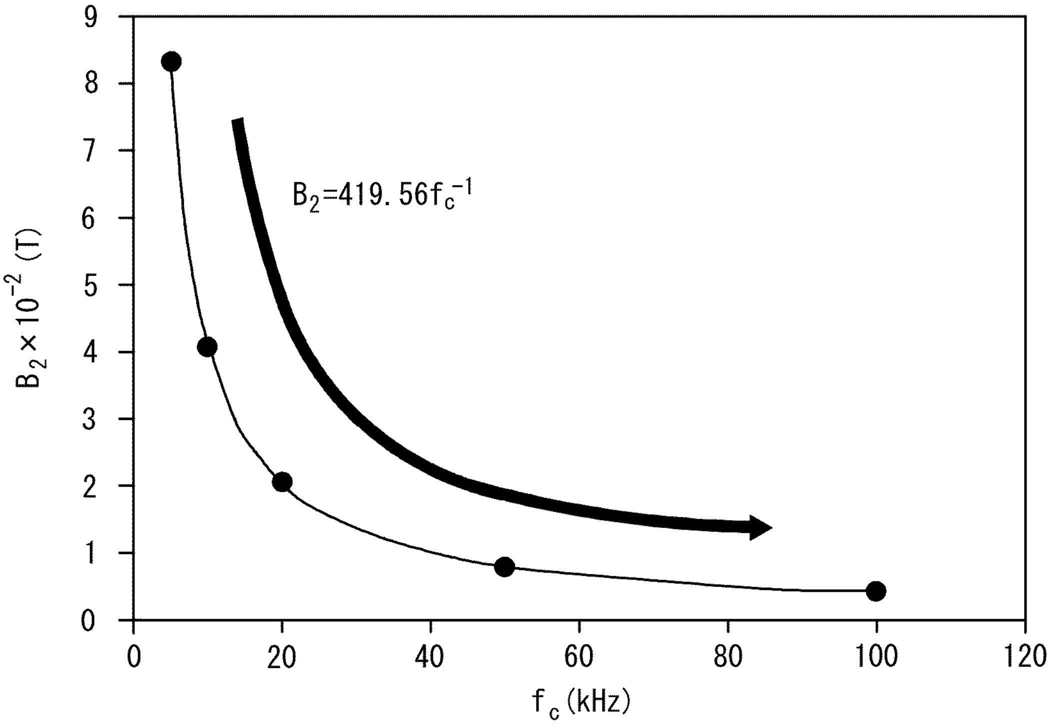
9
8
7
6
5
4
3
2
1
0
B2 × 10^-2 (T)
B2=419. 56fc^-1
0
20
40
60
80
100
120
f c (kHz)

FIG. 10

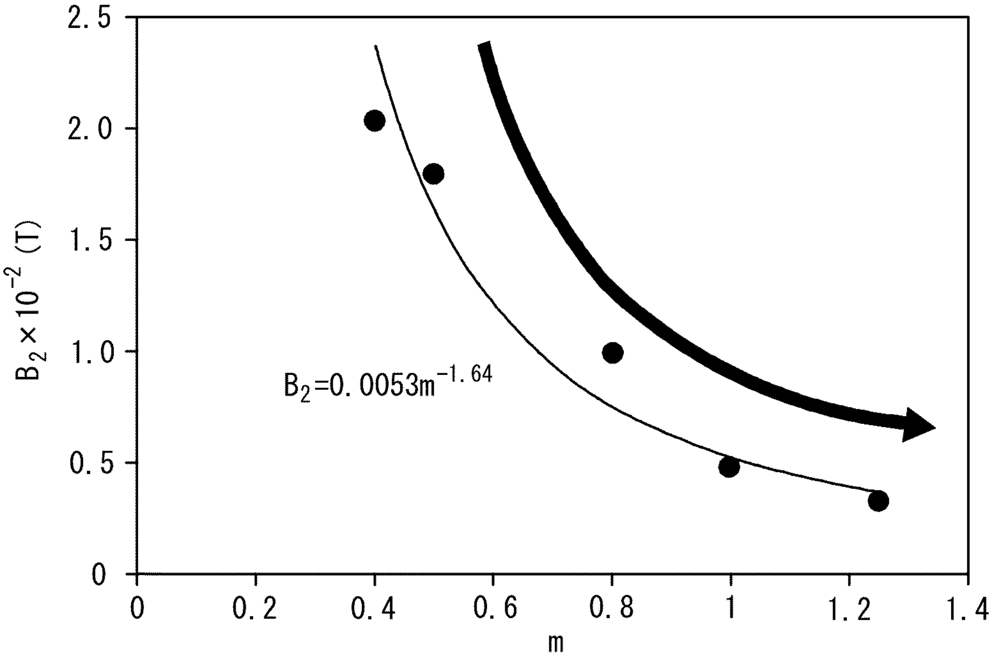
2.5
2.0
1.5
1.0
0.5
0
$B_2 \times 10^{-2}$ (T)
$B_2=0.0053m^{-1.64}$
0
0.2
0.4
0.6
0.8
1
1.2
1.4
m

FIG. 11

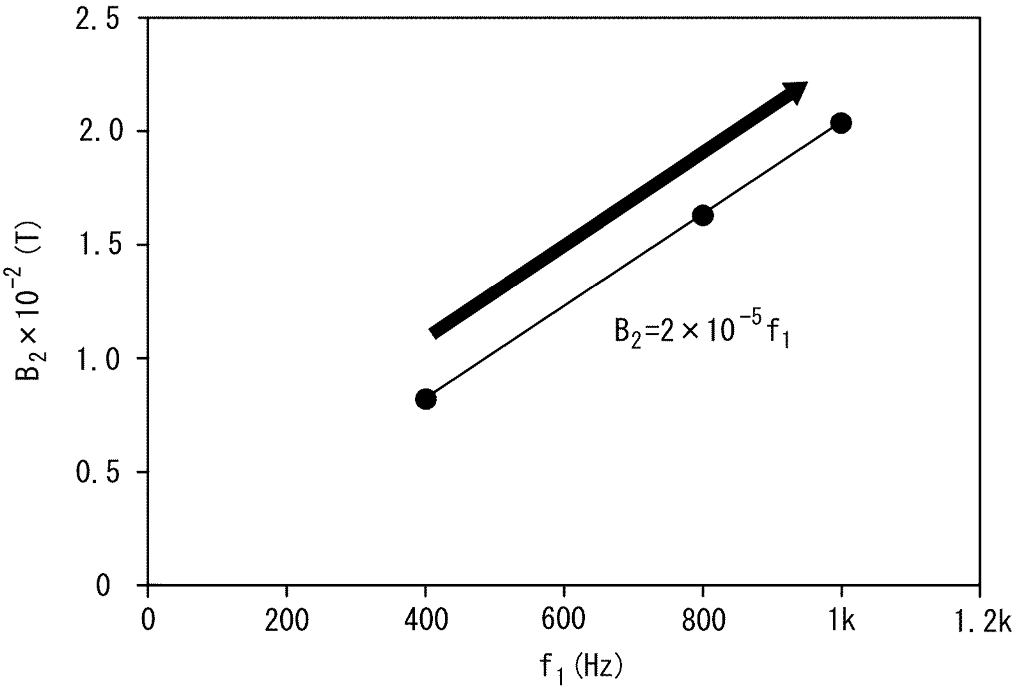
2.5
2.0
1.5
1.0
0.5
0
$B_2 \times 10^{-2}$ (T)
$B_2=2 \times 10^{-5} f_1$
0
200
400
600
800
1k
1.2k
$f_1$ (Hz)

FIG. 12

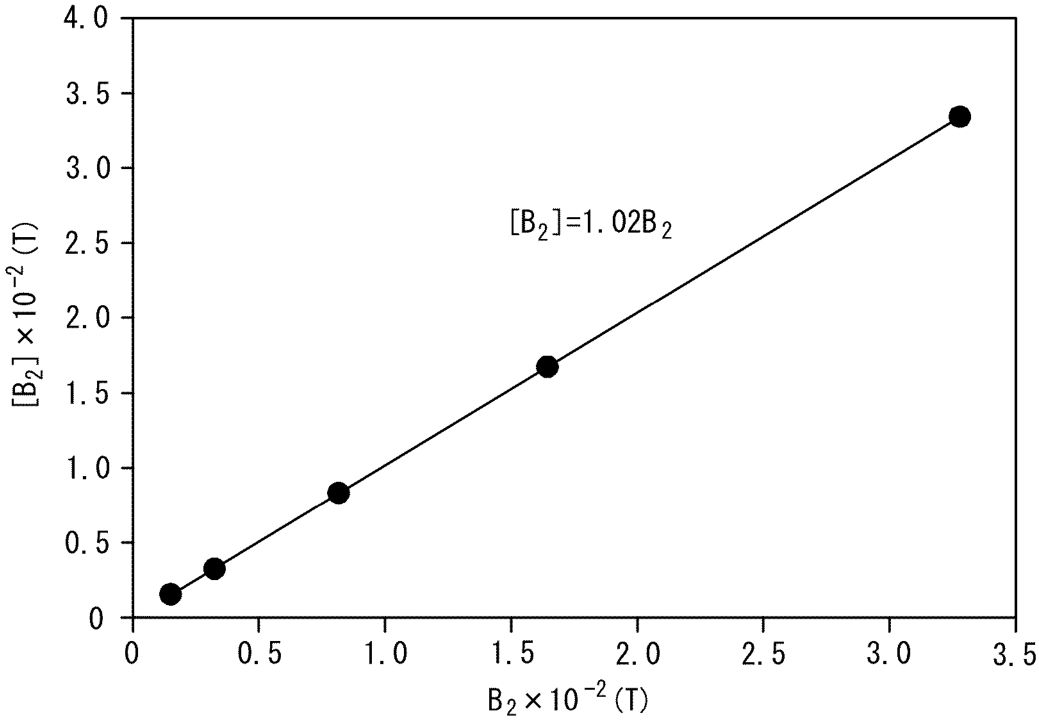


FIG. 13

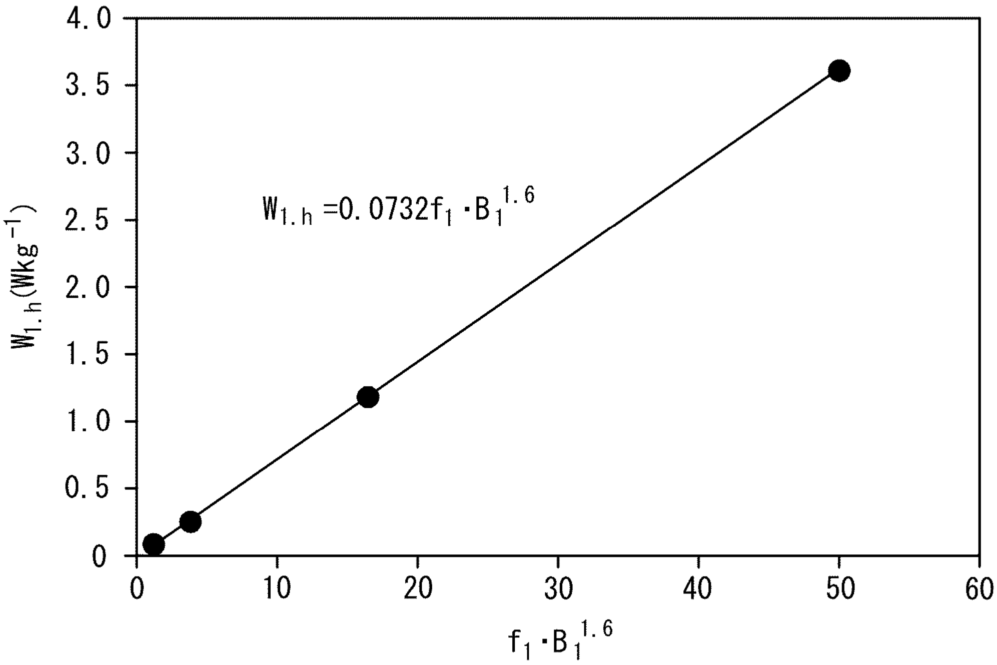

FIG. 14

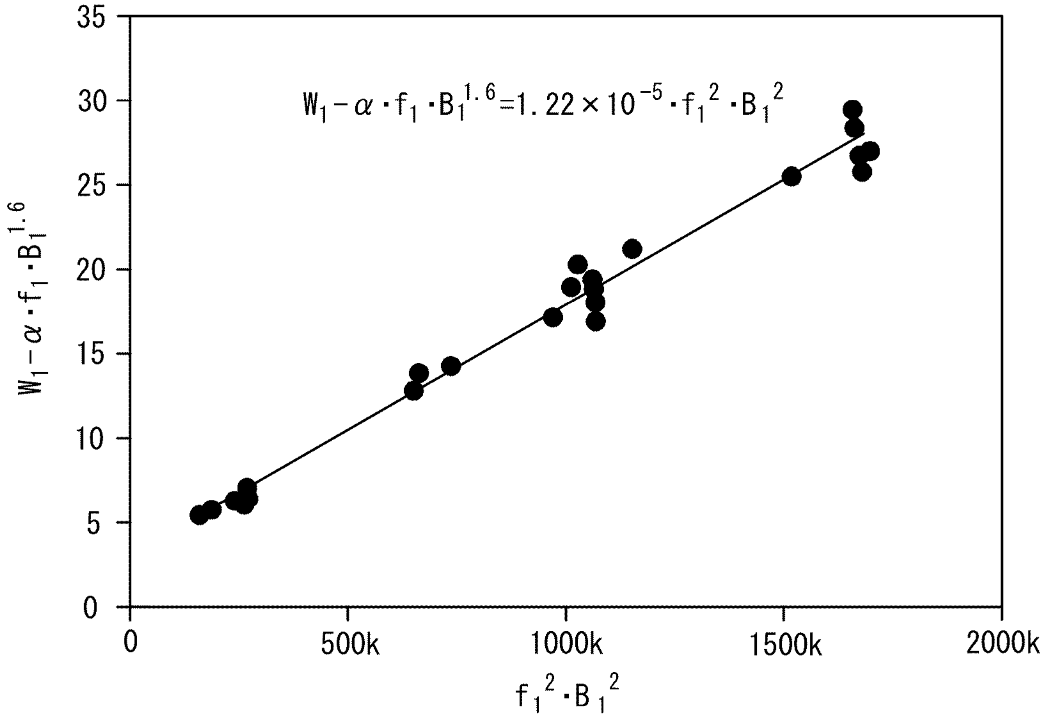
35
30
25
20
15
10
5
0
0
500k
1000k
1500k
2000k
W1−α・f1・B1^1.6=1.22×10^−5・f1^2・B1^2
W1−α・f1・B1^1.6
f1^2・B1^2

FIG. 15

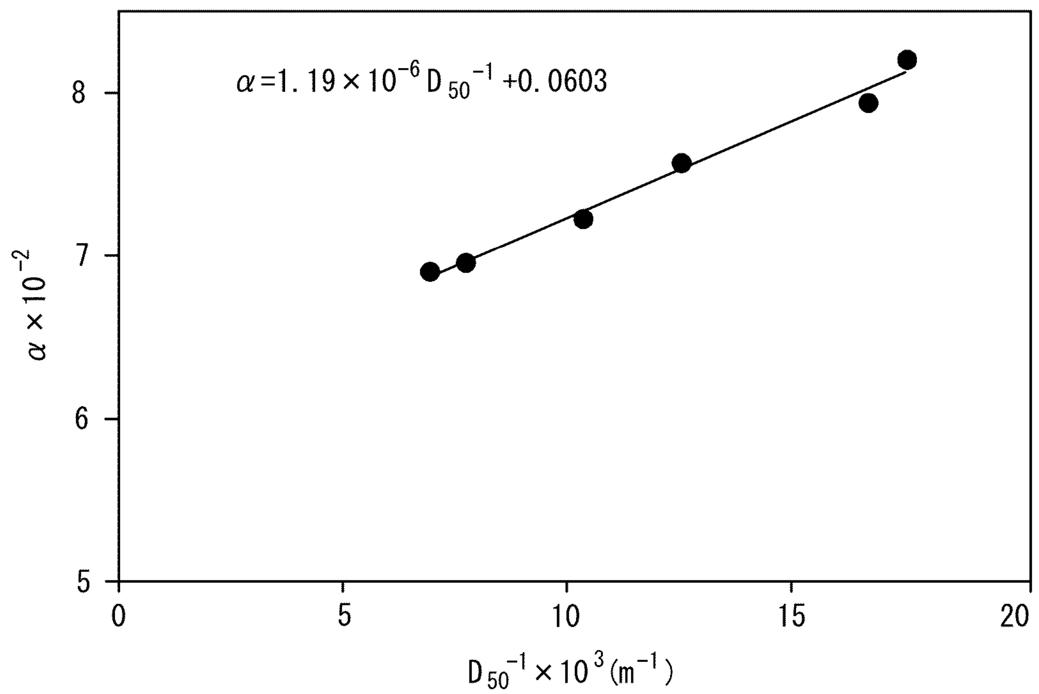
8
7
6
5
0
5
10
15
20
α=1.19×10^−6 D50^−1 +0.0603
α×10^−2
D50^−1×10^3(m^−1)

FIG. 16

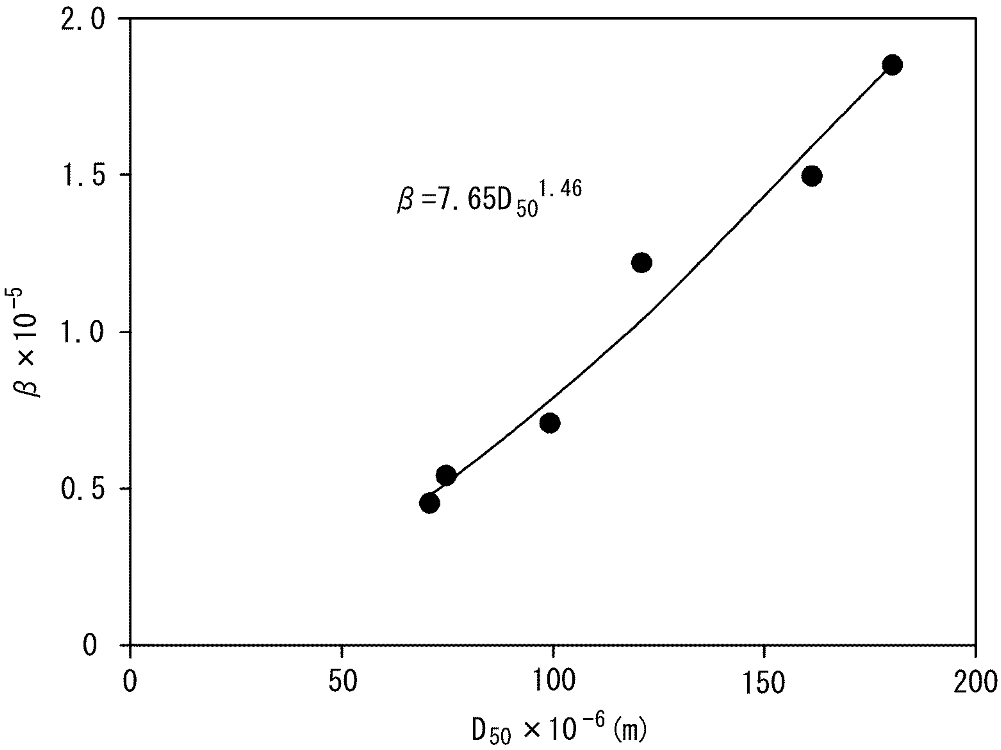
2.0
1.5
1.0
0.5
0
β×10⁻⁵
β=7.65D50^1.46
0
50
100
150
200
D50×10⁻⁶(m)

FIG. 17

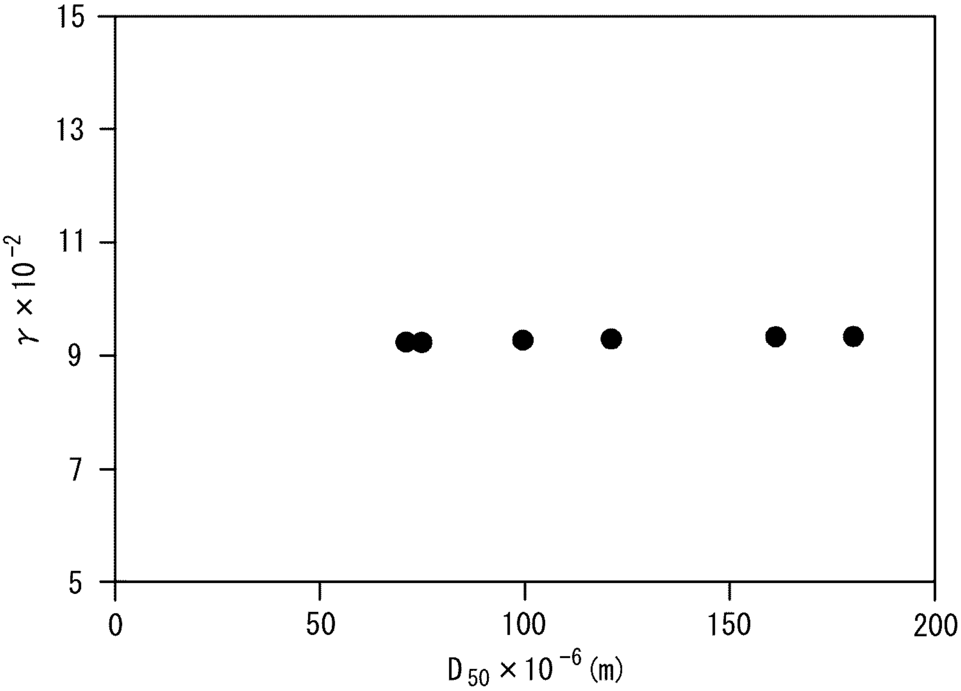
15
13
11
9
7
5
γ×10⁻²
0
50
100
150
200
D50×10⁻⁶(m)

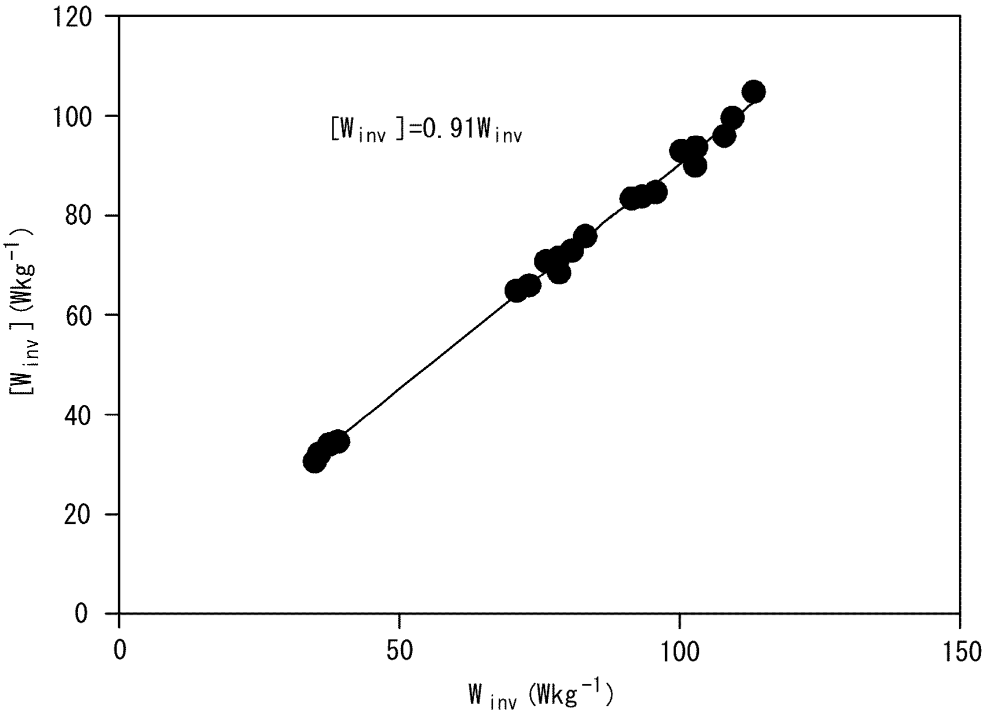

[W inv ]=0. 91W inv
[W inv ] (Wkg-1)
W inv (Wkg-1)
0
20
40
60
80
100
120
0
50
100
150


FIG. 18

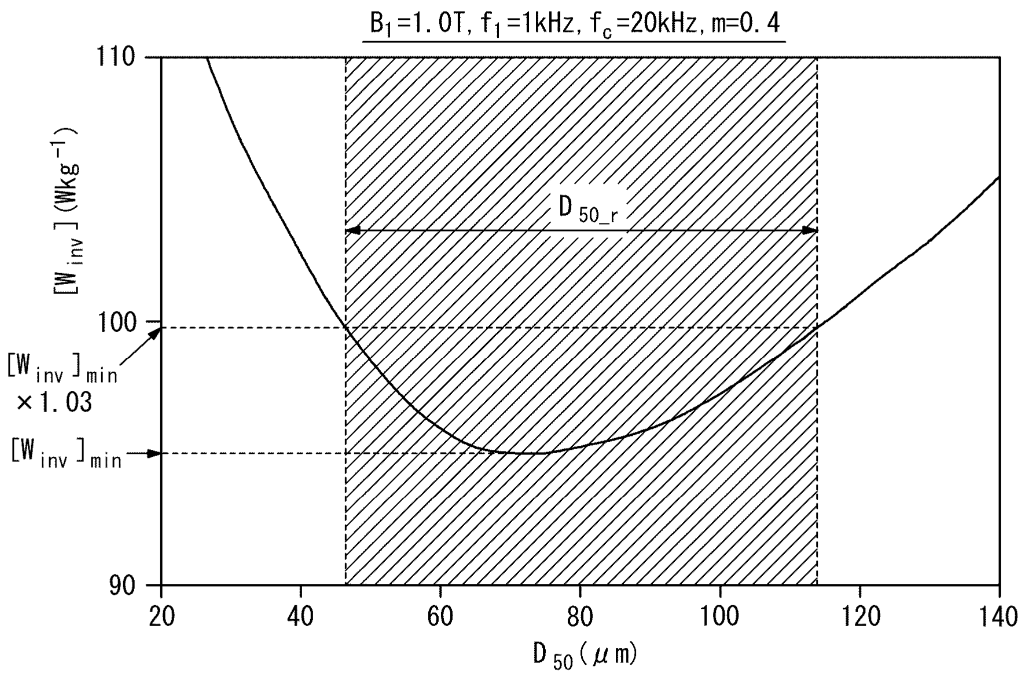

B1=1. 0T, f1=1kHz, fc=20kHz, m=0. 4
[W inv ] (Wkg-1)
D 50_r
[W inv ]min
× 1. 03
[W inv ]min
D 50 (μm)
90
100
110
20
40
60
80
100
120
140


FIG. 19

FIG. 20

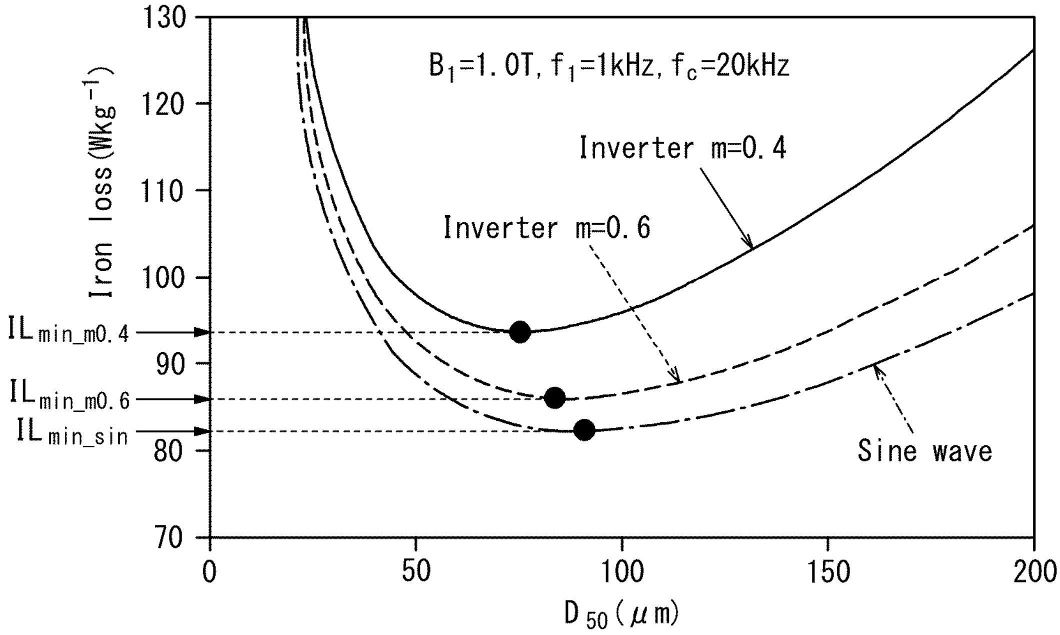
130
120
110
100
90
80
70
Iron loss (Wkg$^{-1}$)
$B_1$=1.0T, $f_1$=1kHz, $f_c$=20kHz
Inverter m=0.4
Inverter m=0.6
Sine wave
$IL_{min_m0.4}$
$IL_{min_m0.6}$
$IL_{min_sin}$
0
50
100
150
200
$D_{50}$ (μm)

FIG. 21

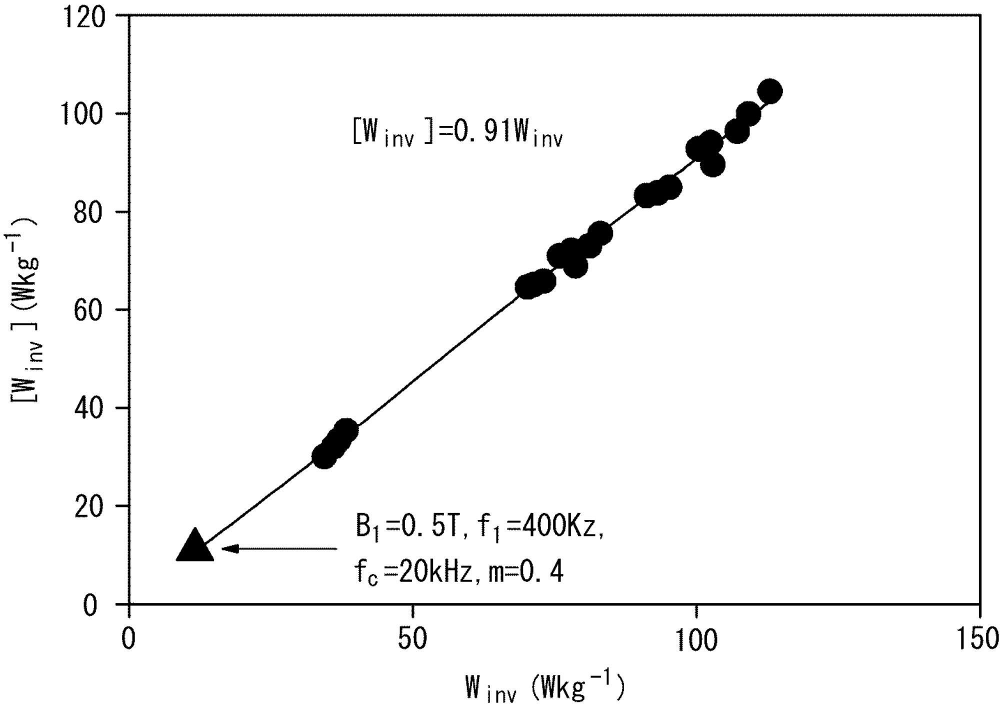
120
100
80
60
40
20
0
[$W_{inv}$] (Wkg$^{-1}$)
[$W_{inv}$]=0.91$W_{inv}$
$B_1$=0.5T, $f_1$=400Kz,
$f_c$=20kHz, m=0.4
0
50
100
150
$W_{inv}$ (Wkg$^{-1}$)

FIG. 22

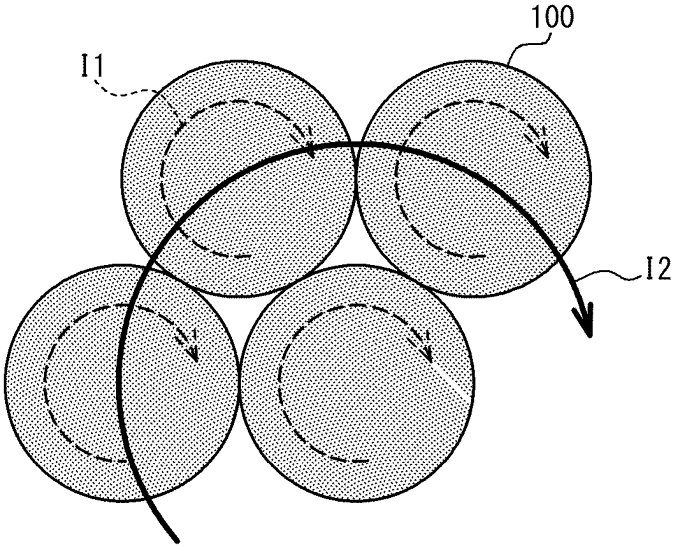


FIG. 23

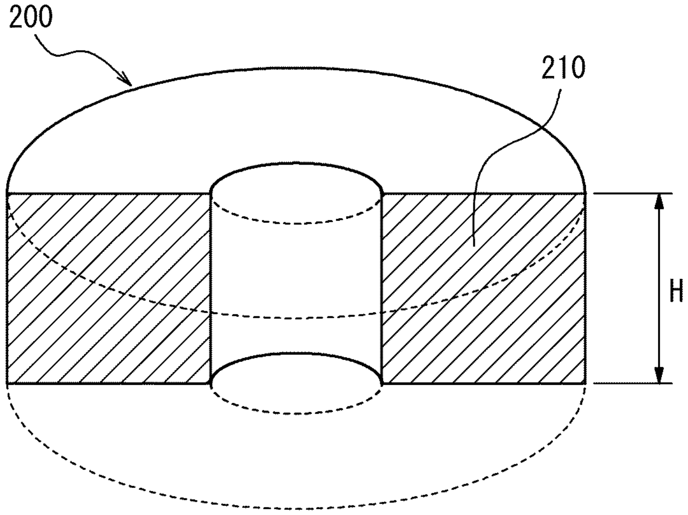

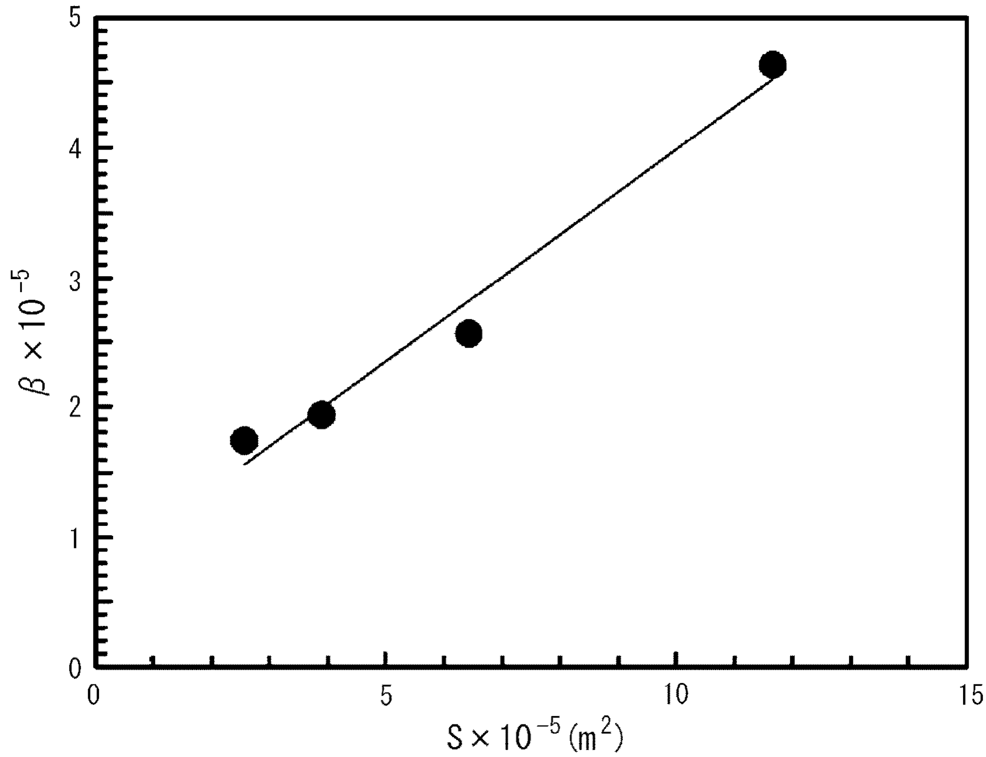


FIG. 24

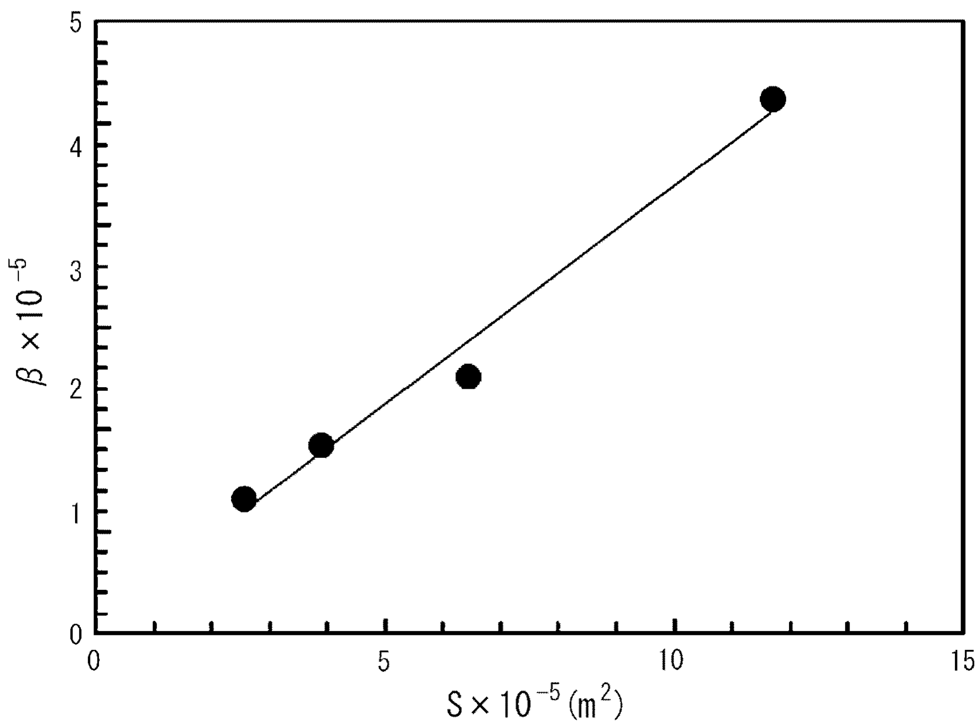


FIG. 25

FIG. 26

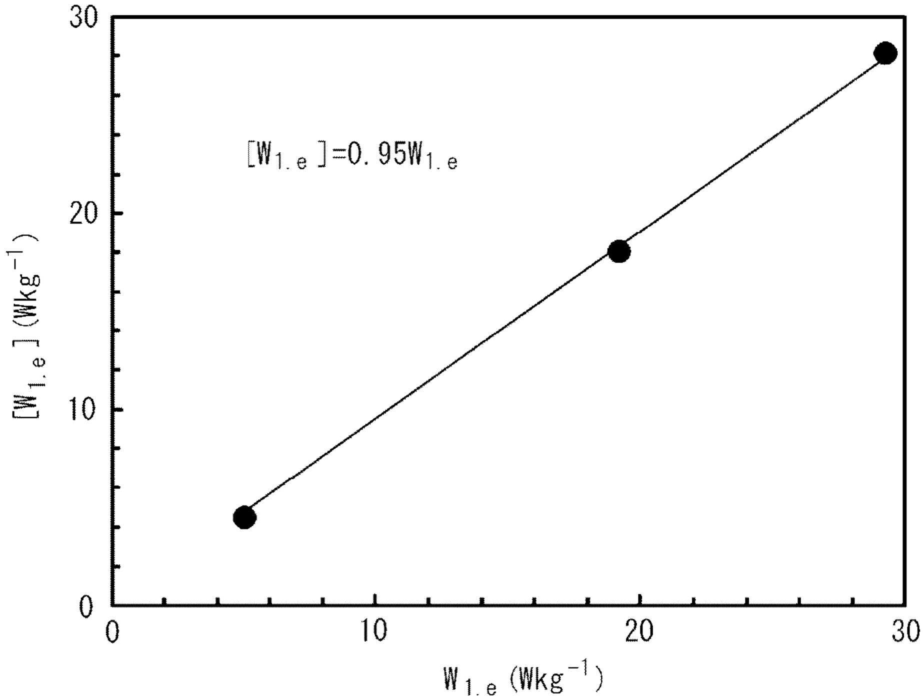
30
20
10
0
0
10
20
30
[W1.e ]=0. 95W1.e
[W1.e ] (Wkg^-1)
W1.e (Wkg^-1)

FIG. 27

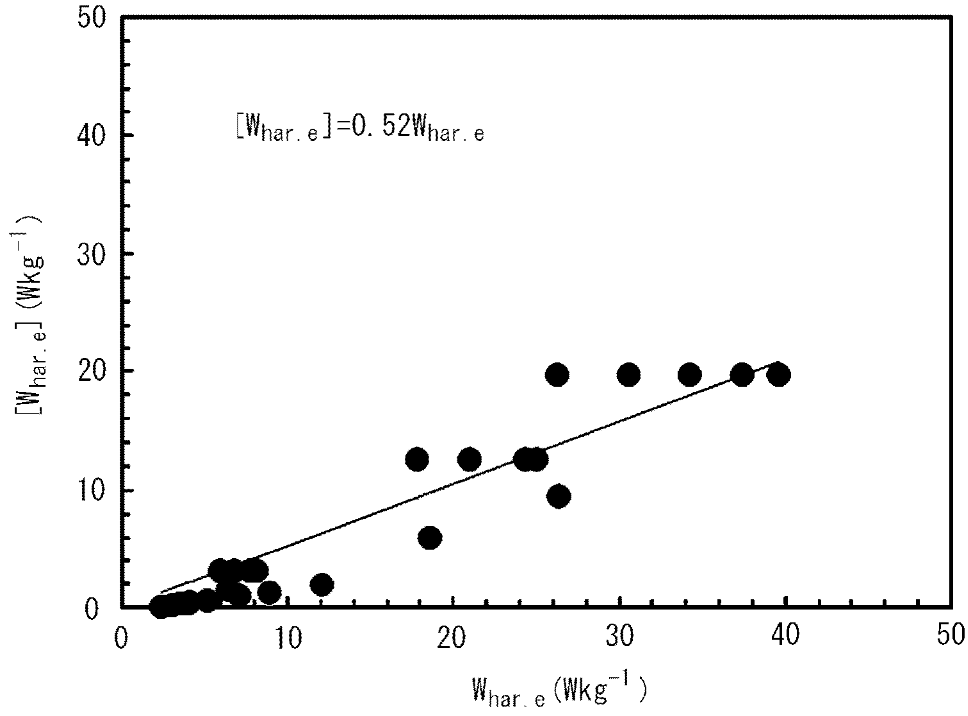
50
40
30
20
10
0
0
10
20
30
40
50
[Whar.e]=0. 52Whar.e
[Whar.e] (Wkg^-1)
Whar.e (Wkg^-1)

FIG. 28

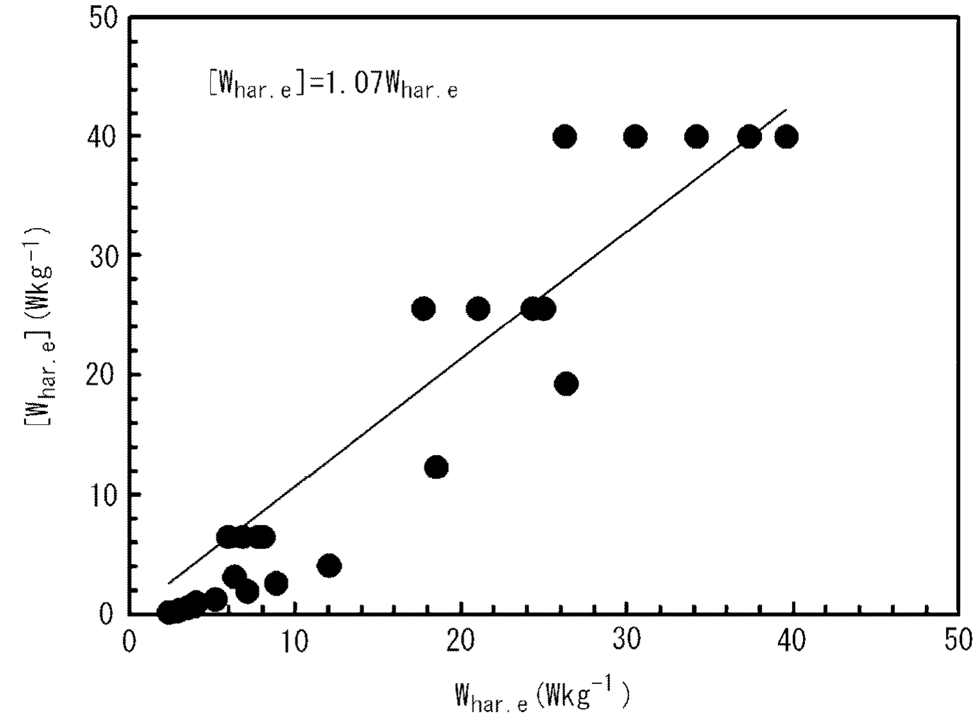
[$W_{har.e}$]=1.07$W_{har.e}$
[$W_{har.e}$] ($Wkg^{-1}$)
$W_{har.e}$ ($Wkg^{-1}$)
0
10
20
30
40
50

FIG. 29

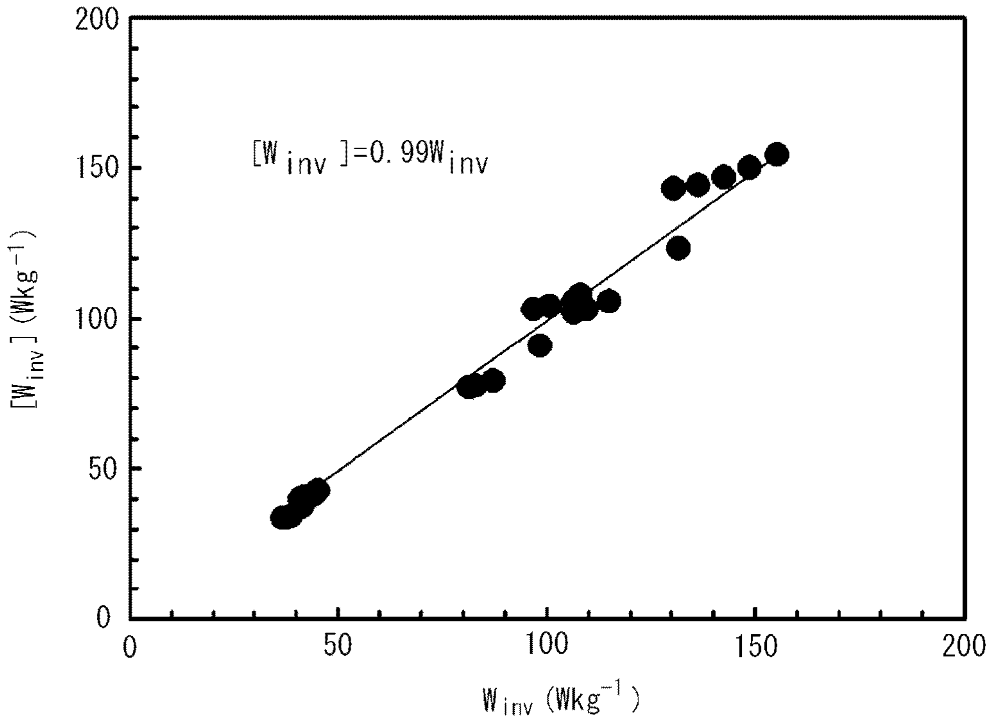
[$W_{inv}$]=0.99$W_{inv}$
[$W_{inv}$] ($Wkg^{-1}$)
$W_{inv}$ ($Wkg^{-1}$)
0
50
100
150
200

## INTERNATIONAL SEARCH REPORT

International application No.
**PCT/JP2024/020252**

**A. CLASSIFICATION OF SUBJECT MATTER**

***G06F 30/20***(2020.01)i
FI: G06F30/20

According to International Patent Classification (IPC) or to both national classification and IPC

**B. FIELDS SEARCHED**

Minimum documentation searched (classification system followed by classification symbols)

G06F30/00-30/398; G01R33/12-33/18; G06Q50/04

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

Google Scholar

**C. DOCUMENTS CONSIDERED TO BE RELEVANT**

| Category* | Citation of document, with indication, where appropriate, of the relevant passages | Relevant to claim No. |
|---|---|---|
| X | JP 2019-204269 A (NIPPON STEEL CORPORATION) 28 November 2019 (2019-11-28) paragraphs [0016]-[0057] | 1,9 |
| Y | | 5, 7, 10-12 |
| A | | 2-4, 6, 8 |
| Y | 五十嵐直人　外５名, 車載用リアクトルの小型化を可能にした純鉄系圧粉コア, ＳＥＩテクニカルレビュー [online], January 2015, no. 186, pp. 92-97, [retrieved on 12 July 2024], Retrieved from the Internet <URL:https://sei.co.jp/technology/tr/bn186/pdf/sei10848.pdf>, (IGARASHI, Naoto et al. Pure Iron Based Soft Magnetic Composite Core That Enables Downsizing Automotive Reactors. SEI technical review.) p. 93, right column, line 28 to p. 94, left column, line 5 | 5, 7, 10-12 |
| A | JP 2012-026960 A (FUJI ELECTRIC CO., LTD.) 09 February 2012 (2012-02-09) claims | 1-12 |

☐ Further documents are listed in the continuation of Box C.　☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 July 2024** | **06 August 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/020252**

| Patent document cited in search report | Publication date (day/month/year) | Patent family member(s) | Publication date (day/month/year) |
|---|---|---|---|
| JP 2019-204269 A | 28 November 2019 | (Family: none) | |
| JP 2012-026960 A | 09 February 2012 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2012026960 A


### Non-patent literature cited in the description


- **G. BERTOTTI**. *IEEE TRANSACTIONS ON MAGNETICS*, 1988, vol. 24 (1), 621-630